(19) Europäisches Patentamt / European Patent Office / Office européen des brevets

(11) **EP 3 858 310 B1**

(12) **EUROPÄISCHE PATENTSCHRIFT**

(45) Veröffentlichungstag und Bekanntmachung des Hinweises auf die Patenterteilung:
**14.09.2022 Patentblatt 2022/37**

(21) Anmeldenummer: **20154937.5**

(22) Anmeldetag: **31.01.2020**

(51) Internationale Patentklassifikation (IPC):
**A61F 13/15** (2006.01)    **A61F 13/49** (2006.01)
**A61F 13/551** (2006.01)    **A61F 13/56** (2006.01)

(52) Gemeinsame Patentklassifikation (CPC):
**A61F 13/55115; A61F 13/15747; A61F 13/49058; A61F 13/5633;** A61F 2013/49077

(54) **INKONTINENZWEGWERFWINDEL MIT ASYMMETRISCHER FALTUNG DER HINTEREN SEITENABSCHNITTE**

DISPOSABLE INCONTINENCE NAPPY WITH ASYMMETRIC FOLDING OF THE REAR SIDE PORTIONS

COUCHE POUR INCONTINENCE JETABLE À PLIAGE ASYMÉTRIQUE DES PARTIES LATÉRALES ARRIÈRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**04.08.2021 Patentblatt 2021/31**

(73) Patentinhaber: **Paul Hartmann AG**
**89522 Heidenheim (DE)**

(72) Erfinder:
• **Thirion, Joel**
  **67220 Saint Maurice (FR)**
• **Jaeger, Noel**
  **67650 Dieffenthal (FR)**

• **Oziau, Francois Marie**
  **67220 Neubois (FR)**
• **Möhlmann, Jochen**
  **67220 Thanvillé (FR)**

(74) Vertreter: **Oltmann, Eckhard et al**
**Paul Hartmann AG**
**Patents & Licensing SM-PL**
**Paul-Hartmann-Straße 12**
**89522 Heidenheim (DE)**

(56) Entgegenhaltungen:
WO-A1-97/33815          DE-A1-102015 226 815
DE-A1-102018 104 539

**Beschreibung**

Die vorliegende Erfindung betrifft gefaltete Inkontinenzwegwerfwindeln mit Seitenabschnitten für die Aufnahme von Körperausscheidungen für die bevorzugte Verwendung durch Erwachsene.

Derartige Inkontinenzwegwerfwindeln sind bekannt und weisen häufig einen Hauptteil (Chassis), bestehend aus einem Vorderbereich, einem Rückenbereich und einem in Längsrichtung der Windel dazwischen liegenden und bei Gebrauch zwischen den Beinen eines Benutzers zu liegen kommenden Schrittbereich auf, wobei der Hauptteil meist bereits einen Flüssigkeiten absorbierenden Saugkörper umfasst, und mit an hinteren seitlichen Längsrändern des Rückenbereichs beidseitig angefügten, voneinander separaten und vorzugsweise mit Verschlussmitteln versehenen hinteren Seitenabschnitten, sogenannten Windelohren, welche sich in Querrichtung der Windel über die hinteren seitlichen Längsränder des Hauptteils hinaus erstrecken und den Vorderbereich und den Rückenbereich im angelegten Zustand der Windel miteinander verbinden.

Dieser offene Windel-Typ wird, im Gegensatz zu "pant-type", üblicherweise derart an den Körper angelegt, dass die hinteren Seitenabschnitte der Windel in je nach anatomischen Gegebenheiten unterschiedlich starker Überlappung mit einer Außenseite des Vorderbereichs der Windel gebracht und dort lösbar insbesondere mittels klebenden oder mechanischen Verschlussmitteln angeheftet werden. Eine derartige Windel ist beispielsweise aus WO2017/140604A1 bekannt.

Inkontinenzwegwerfwindeln werden üblicherweise während des Herstellungsprozesses mehrfach gefaltet, um eine kompakte Anordnung zu erreichen, wodurch die Verpackung und der Transport der Inkontinenzwegwerfwindeln erleichtert wird. Insbesondere bei der typischen Herstellung in schnell laufenden Maschinen können dabei abstehende Bauteile wie beispielsweise seitlich überstehende Seitenabschnitte leicht beschädigt werden. Das führt häufig dazu, dass die Produkte beim Auspacken durch den Benutzer unansehnlich erscheinen. Zudem kann die Funktion von an den Seitenabschnitten angebrachten Verschlussmitteln durch Knicke beeinträchtigt sein.

Beispielsweise offenbart WO2005/110321A1 einen absorbierenden Artikel, der derart gefaltet ist, dass ein Teil eines jeweiligen Windelohres nach außen überstehend zu liegen kommt und leicht ergreifbar ist. Überstehende Teile des Windelohres könnten jedoch in nachfolgenden Herstellungsschritten und beim Verpacken der Artikel geknickt werden, was sich insbesondere auf die Funktion von in diesem Bereich angebrachten Verschlussmitteln nachteilig auswirken kann.

Daher besteht ein Bedarf an Inkontinenzwegwerfwindeln, die prozesssicher bereitstellbar sind und eine erleichterte Handhabung beim Entfalten und Anlegen der Windel aufweisen.

Der vorliegenden Erfindung liegt die Aufgabe zu Grunde, verbesserte Inkontinenzwegwerfwindeln bereitzustellen, deren Seitenabschnitte weniger herstellungsbedingte Beschädigungen aufweisen, die bei der Entnahme aus der Verpackung ansehnlicher sind und deren Funktionalität unbeeinträchtigt ist.

Weiterhin liegt der Erfindung die Aufgabe zu Grunde, verbesserte Inkontinenzwegwerfwindeln bereitzustellen, deren Seitenabschnitte beim Entfalten leichter ergreifbar und einfacher handhabbar sind.

Diese Aufgaben werden gelöst durch eine Inkontinenzwegwerfwindel mit einer Längsrichtung, einer Querrichtung, einer Längsmittelachse LM, und einem Hauptteil umfassend einen Saugkörper, wobei der Hauptteil einen Vorderbereich mit einem ersten und einem in Querrichtung gegenüber angeordneten zweiten vorderen seitlichen Längsrand und einen Rückenbereich mit einem ersten und einem in Querrichtung gegenüber angeordneten zweiten hinteren seitlichen Längsrand aufweist, und wobei der Hauptteil einen zwischen dem Vorderbereich und dem Rückenbereich angeordneten Schrittbereich aufweist, und wobei der Hauptteil einen ersten hinteren Seitenabschnitt und einen zweiten hinteren Seitenabschnitt aufweist, wobei der erste hintere Seitenabschnitt an den ersten hinteren seitlichen Längsrand angefügt ist und wobei der zweite hintere Seitenabschnitt an den zweiten hinteren seitlichen Längsrand angefügt ist, wobei der erste und der zweite hintere Seitenabschnitt jeweils einen in der Querrichtung elastischen Bereich aufweisen, wobei der erste und der zweite hintere Seitenabschnitt jeweils wenigstens ein Verschlussmittel aufweisen, so dass der erste und der zweite hintere Seitenabschnitt zum Anlegen und Schließen der Inkontinenzwegwerfwindel an einen Benutzer jeweils entlang einer Hüftumfangsrichtung um den Körper des Benutzers herumlegbar und in überlappende Anordnung mit einer Außenseite des Vorderbereiches bringbar sind, an der sie über das jeweilige Verschlussmittel jeweils lösbar anhaftbar sind,

a) wobei der erste hintere Seitenabschnitt an einer Anzahl N1 in der Längsrichtung verlaufender erster Seitenabschnitt-Falzachsen zu einer ersten Faltanordnung des ersten hinteren Seitenabschnitts auf sich selbst gefaltet ist,
b) wobei der zweite hintere Seitenabschnitt an einer Anzahl N2 in der Längsrichtung verlaufender zweiter Seitenabschnitt-Falzachsen zu einer ersten Faltanordnung des zweiten hinteren Seitenabschnitts auf sich selbst gefaltet ist,
c) wobei die erste Faltanordnung des ersten hinteren Seitenabschnitts an einer Anzahl N3 von in der Längsrichtung verlaufenden ersten Produktfalzachsen nach innen auf eine körperzugewandte Oberseite des Hauptteils gefaltet ist zu einer zweiten Faltanordnung,
d) wobei die erste Faltanordnung des zweiten hinteren Seitenabschnitts an einer Anzahl N4 von in der Längsrichtung verlaufenden zweiten Produktfalzachsen nach innen auf die körperzugewandte Oberseite des Hauptteils gefaltet ist zu einer dritten Faltanordnung,
e) wobei gilt N3<N4.

Die Faltung an vordefinierten ersten und zweiten Seitenabschnitt-Falzachsen ermöglicht es auf vorteilhafte Weise, die Gefahr von Beschädigungen durch Knicke in dem jeweils wenigstens einen Verschlussmittel des jeweiligen ersten und zweiten hinteren Seitenabschnitts bei der Ausbildung der jeweiligen ersten Faltanordnung zu reduzieren.

Die Faltung nach innen auf die körperzugewandte Oberseite des Hauptteils verbessert weiter eine vor Beschädigungen geschützte Anordnung des ersten und zweiten hinteren Seitenabschnitts und insbesondere der jeweiligen Verschlussmittel.

Außerdem erhöht eine mindestens Zwei- oder Mehrlagigkeit der ersten Faltanordnung des ersten und zweiten hinteren Seitenabschnitts die Stabilität der jeweiligen ersten Faltanordnung, was sich positiv auf die Prozesssicherheit bei der Ausbildung der zweiten und dritten Faltanordnung auswirkt.

Im Rahmen der vorliegenden Erfindung sind als erste oder zweite Seitenabschnitt-Falzachsen diejenigen Falzachsen zu verstehen, welche innerhalb des jeweiligen ersten oder zweiten hinteren Seitenabschnitts in der Längsrichtung der Inkontinenzwegwerfwindel verlaufen, und wobei bei der Faltung entlang dieser Falzachsen eine jeweilige erste Faltanordnung ausgebildet wird, bei der ein jeweiliger erster oder zweiter hinterer Seitenabschnitt nur auf sich selbst gefaltet ist und nicht mit dem Hauptteil in Überlappung gebracht ist. In der ersten Faltanordnung ist der erste und/oder zweite hintere Seitenabschnitt der Inkontinenzwegwerfwindeln vorzugsweise in dieser Konfiguration vorläufig und/oder lösbar fixiert.

Eine Faltung an den ersten oder zweiten Seitenabschnitt-Falzachsen erfolgt in bevorzugter Weise vor einem Fügen des jeweiligen ersten oder zweiten hinteren Seitenabschnitts an den jeweiligen ersten oder zweiten hinteren seitlichen Längsrand des Rückenbereichs. Alternativ kann die Faltung an den ersten oder zweiten Seitenabschnitt-Falzachsen nach dem Fügen des jeweiligen ersten oder zweiten hinteren Seitenabschnitts an den jeweiligen ersten oder zweiten hinteren seitlichen Längsrand des Rückenbereichs erfolgen.

Der erste und der zweite hintere Seitenabschnitt der Inkontinenzwegwerfwindel sind derart an den jeweiligen ersten oder zweiten hinteren seitlichen Längsrand des Rückenteils angefügt, dass der erste und zweite hintere Seitenabschnitt in der Querrichtung voneinander beabstandet sind, und in einem aufgefalteten und eben ausgebreiteten Zustand der Inkontinenzwegwerfwindel sich der erste hintere Seitenabschnitt über den ersten hinteren seitlichen Längsrand des Rückenbereichs hinaus und der zweite hintere Seitenabschnitt sich über den zweiten hinteren seitlichen Längsrand des Rückenbereichs hinaus erstreckt. Vorzugsweise ist der erste und zweite hintere Seitenabschnitt jeweils mit dem Hauptteil überlappend angefügt, wobei der erste und der zweite hintere Seitenabschnitt jeweils innerhalb eines jeweiligen Fügebereiches bevorzugt mittels Ultraschallschweißen mit dem Hauptteil verbunden sind, jedoch ist jedes andere dem Fachmann an sich bekannte und zu dem Zweck geeignete Fügeverfahren, insbesondere Thermoschweißen, Nähen, Siegeln oder Kleben, ebenso möglich.

Bevorzugt verlaufen die ersten und/oder die zweiten Seitenabschnitt-Falzachsen und/oder die ersten und/oder die zweiten Produkt-Falzachsen jeweils entlang der Längsrichtung der Inkontinenzwegwerfwindel, insbesondere parallel zur Längsmittelachse LM.

Eine unterschiedliche Anzahl N3 und N4 von ersten und zweiten Produktfalzachsen ergeben in vorteilhafter Weise eine nicht symmetrische Ausbildung und damit eine unterschiedliche Öffenbarkeit der zweiten und dritten Faltanordnung. Durch die höhere Anzahl N4 an zweiten Produkt-Falzachsen weist die dritte Faltanordnung des zweiten hinteren Seitenabschnitts eine größere Anzahl an Lagen in einer Dickenrichtung, und dadurch insbesondere eine höhere Stabilität auf als die zweite Faltanordnung, wodurch der Benutzer beim Auffalten der Windel die dritte Faltanordnung leichter ergreifen und/oder halten kann.

Ein weiterer Vorteil hierbei ist, dass die dritte Faltanordnung einen tastbaren Unterschied zu der dritten Faltanordnung aufweist und dadurch eine Orientierungshilfe beim Anlegen der Windel bietet, insbesondere bei eingeschränkten Sichtverhältnissen wie beispielsweise in der Nacht, bei Dunkelheit oder bei der Benutzung durch in Ihrem Sehvermögen eingeschränkten Benutzern.

Die zweite Faltanordnung hingegen hat den Vorteil einer einfacheren Entfaltbarkeit im Vergleich zur dritten Faltanordnung durch die im Vergleich zur Anzahl N4 an zweiten Produktfalzachsen der dritten Faltanordnung geringere Anzahl N3 an ersten Produktfalzachsen.

Vorzugsweise gilt N3≥1, N4≤4, insbesondere N3=1 und N4≥2, wobei insbesondere gilt N4=2.

Durch die relativ geringe Anzahl N3 und N4 an ersten und zweiten Produkt-Falzachsen wird eine leichtere Handhabbarkeit bei der Entfaltung durch den Benutzer weiter verbessert. Außerdem sind dadurch, insbesondere bei N3=1, nur eine minimale Anzahl an Manipulationsschritten, sogenannten Transformationen, des jeweiligen ersten oder zweiten hinteren Seitenabschnitts erforderlich, so dass eine Gefahr für Beschädigungen bei der Ausbildung der zweiten oder dritten Faltanordnung reduziert ist.

Im Rahmen der vorliegenden Erfindung wird das Verständnis unterstellt, dass die Bezeichnung der hinteren Seitenabschnitte als erster oder zweiter Seitenabschnitt zur Unterscheidung der beiden hinteren Seitenabschnitte voneinander dient und dass damit keine Festlegung verbunden ist, ob ein jeweiliger erster oder zweiter hinterer Seitenabschnitt in der Inkontinenzwegwerfwindel linksseitig oder rechtsseitig der Längsmittelachse LM angeordnet ist, das heißt ob ein jeweiliger erster oder zweiter hinterer Seitenabschnitt im Gebrauch um eine linke oder rechte Körperseite eines Benutzers herumlegbar ist.

Es ist denkbar, dass durch eine vorbestimmte linksseitige oder rechtsseitige Anordnung des ersten hinteren Seitenabschnitts und damit der zweiten Faltanordnung, und eine daraus folgende jeweils in der Querrichtung gegenüber angeordnete dritte Faltanordnung spezifisch auf die Anwendung durch links- oder rechtshändige Benutzer abgestimmt werden kann.

Nach einer bevorzugten Ausführungsvariante beträgt die Anzahl N1 der in Längsrichtung verlaufenden ersten Seitenabschnitt-Falzachsen und die Anzahl N2 der in Längsrichtung verlaufenden zweiten Seitenabschnitt-Falzachsen jeweils 1-5, insbesondere 1-4, weiter insbesondere 1-3.

Weiter gilt bevorzugt N1=N2, wobei insbesondere gilt N1=2, wobei weiter insbesondere die erste Faltanordnung des ersten hinteren Seitenabschnitts und die erste Faltanordnung des zweiten hinteren Seitenabschnitts jeweils Z-förmig ausgebildet ist.

Bei N1=N2 ergibt sich in vorteilhafter Weise eine gleichartige Öffenbarkeit der ersten Faltanordnung des ersten und des zweiten hinteren Seitenabschnitts und damit eine leichtere Handhabung bei der Entfaltung durch den Benutzer.

In dem Fall, dass der erste und/oder zweite hintere Seitenabschnitt in der ersten Faltanordnung um eine gerade Anzahl N1 und/oder N2, insbesondere um jeweils zwei erste und/oder zweite Seitenabschnitt-Falzachsen, Z-förmig, jeweils auf sich selbst gefaltet ist, weist ein freies Ende des jeweiligen ersten und/oder zweiten hinteren Seitenabschnitts nach außen, also in die dem jeweiligen anderen ersten oder zweiten hinteren Seitenabschnitt entgegengesetzte Richtung. Dadurch kann das freie Ende des jeweiligen ersten und/oder zweiten hinteren Seitenabschnitts in vorteilhafter Weise beim Entfalten der jeweiligen ersten Faltanordnung vom Benutzer leichter ergriffen werden.

In dem Fall, dass der erste und/oder der zweite hintere Seitenabschnitt in der ersten Faltanordnung um eine ungerade Anzahl N1 oder N2, also um beispielsweise jeweils eine, C-förmig, oder jeweils drei erste und/oder zweite Seitenabschnitt-Falzachsen, M-förmig, jeweils auf sich selbst gefaltet ist, weist das freie Ende des jeweiligen ersten und/oder zweiten hinteren Seitenabschnitts nach innen in Richtung des jeweiligen anderen ersten oder zweiten hinteren Seitenabschnitts. Solchen Falls ist das äußere Ende des gefalteten ersten und/oder zweiten hinteren Seitenabschnitts verschieden vom freien Ende des jeweiligen ersten oder zweiten hinteren Seitenabschnitts.

Bei der Ausbildung der ersten Faltanordnung des ersten hinteren Seitenabschnitts ist der erste hintere Seitenabschnitt an einer innersten, das heißt der Längsmittelachse nächstgelegenen, ersten Seitenabschnitt-Falzachse auf eine körperabgewandte Seite des ersten hinteren Seitenabschnitts eingeschlagen.

Das hat den Vorteil, dass das freie Ende des ersten hinteren Seitenabschnitts in der zweiten Faltanordnung leichter ergreifbar ist.

Alternativ kann bei der Ausbildung der ersten Faltanordnung des ersten hinteren Seitenabschnitts der erste hintere Seitenabschnitt an der innersten, das heißt der Längsmittelachse nächstgelegenen, ersten Seitenabschnitt-Falzachse auf eine körperzugewandte Seite des ersten hinteren Seitenabschnitts eingeschlagen sein.

Bei der Ausbildung der ersten Faltanordnung des zweiten hinteren Seitenabschnitts ist der zweite hintere Seitenabschnitt an einer innersten, das heißt der Längsmittelachse nächstgelegenen, zweiten Seitenabschnitt-Falzachse auf eine körperabgewandte Seite des zweiten hinteren Seitenabschnitts eingeschlagen.

Das hat den Vorteil, dass solchen Falls der zweite hintere Seitenabschnitt in der dritten Faltanordnung leichter entfaltbar ist.

Alternativ kann bei der Ausbildung der ersten Faltanordnung des zweiten hinteren Seitenabschnitts der zweite hintere Seitenabschnitt an der innersten, das heißt der Längsmittelachse nächstgelegenen, zweiten Seitenabschnitt-Falzachse auf eine körperzugewandte Seite des zweiten hinteren Seitenabschnitts eingeschlagen sein.

Vorzugsweise ist die erste Faltanordnung des ersten und des zweiten hinteren Seitenabschnitts jeweils an der jeweiligen innersten, das heißt der Längsmittelachse nächstgelegenen, ersten oder zweiten Seitenabschnitt-Falzachse auf eine körperabgewandte Seite des jeweiligen ersten oder zweiten hinteren Seitenabschnitts eingeschlagen. Das unterstützt in vorteilhafter Weise weiter eine gleichartige Öffenbarkeit der ersten Faltanordnung des ersten und des zweiten hinteren Seitenabschnitts und damit eine leichtere Handhabung bei der Entfaltung durch den Benutzer.

In einer bevorzugten Ausführungsform sind die erste Faltanordnung des ersten und des zweiten hinteren Seitenabschnitts symmetrisch ausgebildet, insbesondere spiegelsymmetrisch zueinander mit der Längsmittelachse LM der Inkontinenzwegwerfwindel als Symmetrieachse.

Das unterstützt auf eine vorteilhafte Weise eine erleichterte Handhabbarkeit durch den Benutzer weiter, da der Benutzer in einer späteren Phase des Anlegeprozesses nicht seitenspezifisch unterschiedliche erste Faltanordnungen zu berücksichtigen braucht.

In einer weiteren bevorzugten Ausführungsvariante ist das wenigstens eine Verschlussmittel des ersten und/oder zweiten hinteren Seitenabschnitts an dem freien Ende des jeweiligen ersten oder zweiten hinteren Seitenabschnitts angeordnet.

In einer bevorzugten Weiterbildung dieser Variante kommt das wenigstens eine Verschlussmittel des ersten und/oder zweiten hinteren Seitenabschnitts in der ersten Faltanordnung außerhalb des jeweiligen ersten oder zweiten hinteren seitlichen Längsrandes des Rückenbereichs, das heißt außerhalb des Hauptteils, zu liegen.

Durch eine solche Anordnung des wenigstens einen Verschlussmittels des jeweiligen ersten und/oder zweiten hinteren Seitenabschnitts wird in vorteilhafter Weise die Gefahr einer herstellungsbedingten Beschädigung des jeweiligen wenigstens einen Verschlussmittels des ersten und/oder zweiten hinteren Seitenabschnitts reduziert, insbesondere während der Faltung an ersten und/oder zweiten Produkt-Falzachsen.

Im Rahmen der vorliegenden Erfindung sind als erste oder zweite Produkt-Falzachsen diejenigen Falzachsen zu verstehen, welche in der Längsrichtung der Inkontinenzwegwerfwindel innerhalb des Hauptteils oder entlang des ersten oder zweiten hinteren seitlichen Längsrandes des Rückenbereiches verlaufen. Darüber hinaus sind auch innerhalb des jeweiligen ersten oder zweiten hinteren Seitenabschnitts verlaufende Falzachsen als erste oder zweite Produkt-Falzachsen zu verstehen, wenn durch die Faltung entlang dieser Falzachsen der jeweilige erste oder zweite hintere Seitenabschnitt zumindest bereichsweise auf den Hauptteil gefaltet und mit dem Hauptteil zumindest bereichsweise in Überlappung gebracht ist.

In einer bevorzugten Ausführungsform verläuft eine in der Querrichtung äußerste in der Längsrichtung verlaufende zweite Produktfalzachse außerhalb des Saugkörpers, insbesondere innerhalb des zweiten hinteren Seitenabschnitts, weiter insbesondere innerhalb des zweiten hinteren Seitenabschnitts und außerhalb der ersten Faltanordnung des zweiten hinteren Seitenabschnitts.

Alternativ kann die in der Querrichtung äußerste in der Längsrichtung verlaufende zweite Produktfalzachse entlang des zweiten hinteren seitlichen Längsrandes des Rückenbereiches verlaufen.

Als weitere Alternative kann die in der Querrichtung äußerste in der Längsrichtung verlaufende zweite Produktfalzachse innerhalb des Hauptteils und außerhalb des Saugkörpers, insbesondere in Querrichtung zwischen dem Saugkörper und dem zweiten hinteren seitlichen Längsrand des Rückenbereichs, weiter insbesondere im Bereich eines von einem Backsheet und einem Topsheet und/oder einem Cuffelement (Cuffvlies) gebildeten und sich in Querrichtung über den Saugkörper hinaus erstreckenden seitlichen Überhanges, weiter insbesondere zwischen einer in Längsrichtung verlaufenden und den Saugkörper an einer in der Querrichtung im Rückenbereich breitesten Stelle seitlich tangierenden imaginären Linie und dem zweiten hinteren seitlichen Längsrand des Rückenbereichs verlaufen, so, dass der Saugkörper nicht die in der Längsrichtung verlaufende zweite Produktfalzachse überfängt.

Als äußerste zweite Produkt-Falzachse ist diejenige zweite Produkt-Falzachse zu verstehen, die im aufgefalteten und eben ausgebreiteten Zustand der Inkontinenzwegwerfwindel den größten Abstand B in Querrichtung zu der Längsmittelachse LM der Inkontinenzwegwerfwindel aufweist.

Dabei wird auf vorteilhafte Weise die Gefahr verringert, dass der Saugkörper bei der Faltung an der äußersten in der Längsrichtung verlaufenden zweiten Produktfalzachse auf sich selbst gefaltet wird, was einem in der Dickenrichtung der Inkontinenzwegwerfwindel starken Aufbau der dritten Faltanordnung und einem weniger ansehnlichen Erscheinungsbild der gefalteten Inkontinenzwegwerfwindel entgegengewirkt. Zusätzlich ist ein weiterer Vorteil, dass die dritte Faltanordnung stabiler ausgebildet ist und sich weniger leicht vorzeitig selbst entfaltet.

Die Dickenrichtung ist im Rahmen der vorliegenden Erfindung als orthogonal zur Längs- und Querrichtung der Inkontinenzwegwerfwindel, gegebenenfalls nach dem Entfalten einer zweiten und/oder dritten Faltanordnung und/oder einer nachfolgend näher beschriebenen vierten Faltanordnung, zu verstehen.

Nach einer bevorzugten Ausführungsform überlappen die zweite und die dritte Faltanordnung zumindest bereichsweise zur Bildung eines Überlappungsbereiches miteinander.

Die Erfinder haben überraschenderweise festgestellt, dass durch die Ausbildung eines Überlappungsbereiches und - ohne an die Theorie gebunden sein zu wollen - die in diesem Überlappungsbereich auftretenden Reibungswirkungen zwischen der zweiten und dritten Faltanordnung, die Stabilität der zweiten und dritten Faltanordnung weiter erhöht wird. Dadurch kann auf vorteilhafte Weise die Gefahr eines zumindest teilweisen Auffaltens der zweiten und dritten Faltanordnung und der damit einhergehenden Beschädigungen des ersten und/oder zweiten hinteren Seitenabschnitts, insbesondere vor oder während der Ausbildung einer nachfolgend näher beschriebenen vierten Faltanordnung, reduziert werden, insbesondere in schnell laufenden Maschinen.

Bevorzugt ist die zweite Faltanordnung in dem Überlappungsbereich zwischen der dritten Faltanordnung und einer körperzugewandten Oberseite des Hauptteils angeordnet.

Diese Anordnung unterstützt weiter die Stabilität der zweiten und dritten Faltanordnung, dabei insbesondere der zweiten Faltanordnung, da Reibungskräfte zwischen der zweiten und dritten Faltanordnung die zweite Faltanordnung auf der körperzugewandten Oberseite des Hauptteils zurückhalten und ein vorzeitiges Auffalten der zweiten und dritten Faltanordnung während des Herstellungsprozesses der Inkontinenzwegwerfwindel hemmen können.

Insbesondere überfängt dabei der zweite hintere Seitenabschnitt zumindest bereichsweise zumindest das freie Ende der ersten Faltanordnung des ersten hinteren Seitenabschnitts, weiter insbesondere zumindest das freie Ende der ersten Faltanordnung des ersten hinteren Seitenabschnitts und zumindest bereichsweise den ersten mindestens zweilagigen Bereich der ersten Faltanordnung des ersten Seitenabschnitts, weiter insbesondere zumindest einen von dem wenigstens einen Verschlussmittel des ersten Seitenabschnitts überfangenen Bereich, weiter insbesondere zumindest die erste Faltanordnung des ersten hinteren Seitenabschnitts.

Die zweite und/oder die dritte Faltanordnung kann vorläufig und/oder lösbar fixiert sein.

Bei der Ausbildung der zweiten und dritten Faltanordnung sind die erste Faltanordnung des ersten hinteren Seitenabschnitts und die erste Faltanordnung des zweiten hinteren Seitenabschnitts nach innen, also auf die körperzugewandte Oberseite des Hauptteils gefaltet. Dadurch wird eine Gefahr des Verschmutzens der flüssigkeitsabsorbierenden Oberseite des Hauptteils vor dem Anlegen der Inkontinenzwegwerfwindel an einen Benutzer verringert.

In einer bevorzugten Ausführungsvariante überfängt zumindest die zweite Faltanordnung die Längsmittelachse LM zumindest bereichsweise.

Weiter bevorzugt überfangen die zweite und die dritte Faltanordnung die Längsmittelachse LM jeweils zumindest bereichsweise.

Im Rahmen der vorliegenden Erfindung verläuft die Längsmittelachse LM entlang der Längsrichtung der Inkontinenzwegwerfwindel und in Querrichtung mittig zu einer nachfolgend näher beschriebenen maximalen Spannweite MS der Inkontinenzwegwerfwindel.

Falls eine erste Seitenabschnittsbreite S1 des ersten und eine zweite Seitenabschnittsbreite S2 des zweiten Seitenabschnitts einen Unterschied von mindestens 3 % aufweisen, verläuft die Längsmittelachse LM mittig zu einer maximalen Quererstreckung des Rückenbereiches des Hauptteils.

Die erste S1 und/oder zweite Seitenabschnittsbreite S2 des jeweiligen ersten oder zweiten hinteren Seitenabschnitts ist im Rahmen der vorliegenden Erfindung zu verstehen als maximale Erstreckung eines jeweiligen ersten oder zweiten hinteren Seitenabschnitts in Querrichtung der Inkontinenzwegwerfwindel über den jeweiligen ersten oder zweiten hinteren seitlichen Längsrand hinaus, gemessen in mm vom jeweiligen ersten oder zweiten hinteren seitlichen Längsrand zum jeweiligen nach außen weisenden freien Ende des jeweiligen ersten oder zweiten hinteren Seitenabschnitts im aufgefalteten, ungedehnten und eben ausgebreiteten Zustand.

In einer bevorzugten Ausführungsform beträgt ein Verhältnis der Erstreckung S1 des ersten hinteren Seitenabschnitts der Inkontinenzwegwerfwindel zu der Erstreckung S2 des zweiten hinteren Seitenabschnitts der Inkontinenzwegwerfwindel 0,9-1,1, insbesondere 1,0.

Bei einer bevorzugten Ausführungsform sind eine jeweilige in der Querrichtung innerste, das heißt der Längsmittelachse LM nächstgelegene in der Längsrichtung verlaufende erste Produktfalzachse und eine jeweilige in der Querrichtung innerste, das heißt der Längsmittelachse LM nächstgelegene in der Längsrichtung verlaufende zweite Produktfalzachse in einem gleichen Abstand A zur Längsmittelachse LM, insbesondere spiegelsymmetrisch zueinander mit der Längsmittelachse LM als Symmetrieachse angeordnet.

Das bewirkt in vorteilhafter Weise, eine weitgehend symmetrische Anordnung des Hauptteils, insbesondere des Saugkörper, und des Vorderbereichs in der Querrichtung der Inkontinenzwegwerfwindel im gefalteten Zustand, und daher eine insgesamt harmonische und ansehnliche Erscheinung der Inkontinenzwegwerfwindel bei der ersten In-die-Handnahme durch den Benutzer.

Der Hauptteil weist in einer weiteren bevorzugten Ausführungsform beidseits zumindest abschnittsweise eine seitliche Auslaufsperre bildende und beidseits entlang der Längsrichtung verlaufende Cuffelemente auf, wobei insbesondere der von der zweiten und dritten Faltanordnung ausgebildete Überlappungsbereich vollständig zwischen den Cuffelementen, mithin in einem Cuffelement-freien Bereich angeordnet ist.

Ein Vorteil dabei ist, dass die Gefahr eines bereichsweisen starken Aufbaus in der Dickenrichtung der Inkontinenzwegwerfwindel reduziert wird, der sich in einem gemeinsam von dem Überlappungsbereich und einem jeweiligen Cuffelement überfangenen Bereich ergeben könnte. Dadurch ergeben sich gleichmäßigere Abmessungen der gefalteten Inkontinenzwegwerfwindel, wodurch die Prozesssicherheit der weiteren Herstellungs- und Verpackungsschritte verbessert wird und die gefaltete Inkontinenzwegwerfwindel ansehnlicher ist.

Weiterhin ergibt sich dabei eine ausgewogenere Materialanordnung und dadurch verpackungstechnische Vorteile.

In einer weiteren bevorzugten Ausführungsform weist die Inkontinenzwegwerfwindel eine maximale Spannweite MS auf, wobei die maximale Spannweite MS 500-1300 mm, insbesondere 600-1200 mm, weiter insbesondere 650-1000 mm, weiter insbesondere 700-900 mm beträgt.

Bei dieser maximalen Spannweite MS ist die vorgeschlagene asymmetrische Faltung des ersten und zweiten hinteren Seitenabschnitts auf vorteilhafte Weise realisierbar.

Weiterhin ergibt sich bei dieser maximalen Spanweite MS ein positiver Effekt auf die räumliche Anordenbarkeit und die Stabilität der zweiten und dritten Faltanordnung des ersten und zweiten hinteren Seitenabschnitts, und damit auf die Ansehnlichkeit und Handhabbarkeit der Inkontinenzwegwerfwindel.

Als maximale Spannweite MS ist zu verstehen die maximale Quererstreckung der Inkontinenzwegwerfwindel gemessen in mm im Rückenbereich der Inkontinenzwegwerfwindel, also im Bereich des ersten und zweiten hinteren Seitenabschnitts.

Vorzugsweise beträgt eine Länge L der Inkontinenzwegwerfwindel 600 mm-1100 mm, insbesondere 650 mm-1050 mm, vorzugsweise 700 mm-1000 mm.

Die Länge L der Inkontinenzwegwerfwindel entspricht der maximalen Erstreckung der Inkontinenzwegwerfwindel in der Längsrichtung im aufgefalteten und eben ausgebreiteten Zustand.

Bevorzugt weist die Inkontinenzwegwerfwindel eine Endfaltbreite EF auf, wobei ein Verhältnis EF zu MS insbesondere 0,15-0,35, weiter insbesondere 0,17-0,30 beträgt.

Bei diesem Verhältnis EF zu MS kann eine vorteilhafte geometrische Anordnung der ersten und zweiten Seitenabschnitt-Falzachsen und der ersten und zweiten Produktfalzachsen realisiert werden, bei der die Gefahr von Knicken in den jeweiligen Verschlussmitteln leichter verringert werden kann und wodurch die jeweiligen ersten, zweiten und dritten Faltanordnungen weniger häufig im Herstellungsprozess vorzeitig auffalten und beschädigt werden können.

Die Endfaltbreite EF entspricht einer maximalen Quererstreckung der Inkontinenzwegwerfwindel mit erster und zweiter und dritter Faltanordnung.

Die Endfaltbreite EF entspricht der Quererstreckung der Inkontinenzwegwerfwindel in dem Zustand der ersten In-die-Handnahme durch den Benutzer, also insbesondere in dem herstellerseitig vorgesehenen Zustand in dem die Inkontinenzwegwerfwindel vom Benutzer aus einer Verpackung entnommen wird und/oder bevor der Benutzer die Inkontinenzwegwerfwindel zum Zwecke des Anlegens auffaltet.

Nach einer bevorzugten Ausführungsform weist die Inkontinenzwegwerfwindel mit erster Faltanordnung eine Spannweite GF1 auf, wobei ein Verhältnis GF1 zu MS 0,50-0,85, insbesondere 0,55-0,75, weiter insbesondere 0,60-0,70 beträgt.

Die Spannweite GF1 der Inkontinenzwegwerfwindel mit erster Faltanordnung ist die maximale Erstreckung der Inkontinenzwegwerfwindel in Querrichtung im Bereich der auf sich selbst gefalteten ersten und zweiten hinteren Seitenabschnitte, also von einem äußeren Ende eines auf sich selbst gefalteten ersten hinteren Seitenabschnittes zu dem äußeren Ende eines auf sich selbst gefalteten zweiten hinteren Seitenabschnittes gemessen in mm.

Dieses Verhältnis GF1 zu MS bietet den Vorteil, dass die zweite und dritte Faltanordnung mit einer relativ geringen jeweiligen Anzahl N2 oder N3 von jeweiligen ersten oder zweiten Produkt-Falzachsen realisiert werden können, so dass dabei die Gefahr von Beschädigungen des ersten und/oder zweiten Seitenabschnitts weiter auf ein Mindestmaß reduziert sowie die Entfaltbarkeit erleichtert werden kann.

Bevorzugt weist eine Inkontinenzwegwerfwindel eine vordere Spannweite VS auf. Weiter bevorzugt beträgt ein Verhältnis der maximalen Spannweite MS der Inkontinenzwegwerfwindel zu einer vorderen Spannweite VS der Inkontinenzwegwerfwindel 1,3 - 3,5, insbesondere 1,5 - 3,2, weiter insbesondere 1,8 - 3,0, weiter insbesondere 2,0 - 2,8.

Als vordere Spannweite ist zu verstehen eine maximale Quererstreckung der Inkontinenzwegwerfwindel im aufgefalteten, eben ausgebreiteten und ungedehnten Zustand gemessen in mm im Vorderbereich des Hauptteils der Inkontinenzwegwerfwindel. Vorzugsweise sind an den Vorderbereich des Hauptteils keine Seitenabschnitte angefügt. Solchen Falls entspricht die vordere Spannweite einer vorderen Hauptteilbreite.

Nach einer bevorzugten Ausführungsform ist die Inkontinenzwegwerfwindel um wenigstens eine, insbesondere um wenigstens zwei, weiter insbesondere um wenigstens drei in der Querrichtung verlaufende Querfalzachsen zu einer vierten Faltanordnung gefaltet, wobei keine der Querfalzachsen durch den ersten oder zweiten hinteren Seitenabschnitt verläuft, insbesondere keine der Querfalzachsen durch die jeweiligen Verschlussmittel verläuft.

Das hat den Vorteil, dass die Struktur und/oder die Funktion der Verschlussmittel und/oder der hinteren Seitenabschnitte nicht durch bei einer Faltung entstehende Knicke beeinträchtigt wird.

Die vierte Faltanordnung ist so zu verstehen, dass die Inkontinenzwegwerfwindel, die bereits in Längsrichtung beidseits um jeweils mindestens eine erste und/oder zweite Seitenabschnitt-Falzachse und/oder um jeweils mindestens eine Produktfalzachse vorgefaltet ist, vorzugsweise zusätzlich um eine oder mehr als eine Querfalzachse gefaltet ist.

Die Inkontinenzwegwerfwindel ist in einer vierten Faltanordnung vorzugsweise so um eine oder mehr als eine Querfalzachse gefaltet, dass der Hauptteil der Inkontinenzwegwerfwindel auf sich selbst gefaltet ist, derart, dass eine Oberseite des Hauptteils zumindest bereichsweise direkt oder mittelbar auf sich selbst zu liegen kommt.

Die Oberseite des Hauptteils kann die im Gebrauchszustand körperabgewandte Außenseite, insbesondere jedoch die körperzugewandte Oberseite des Hauptteils sein.

Bevorzugt weisen der erste und/oder der zweite hintere Seitenabschnitt an dem jeweiligen in Querrichtung freien Ende einen Anfassbereich auf, wobei weiter bevorzugt der Anfassbereich zumindest eines des ersten oder zweiten hinteren Seitenabschnitts nach dem Entfalten der vierten Faltanordnung durch einen Nutzer ergreifbar ist.

In einer bevorzugten Weiterbildung ist das jeweils wenigstens eine Verschlussmittel des ersten oder zweiten hinteren Seitenabschnitts im aufgefalteten und eben ausgebreiteten Zustand der Inkontinenzwegwerfwindel in einem Bereich eines jeweiligen in der Querrichtung freien Endes des jeweiligen ersten oder zweiten hinteren Seitenabschnitts angeordnet.

Weiter bevorzugt weisen der erste und der zweite hintere Seitenabschnitt im Bereich ihres in Querrichtung freien Endes jeweils wenigstens ein Verschlussmittel, weiter insbesondere genau ein Verschlussmittel auf.

Es handelt sich bei den Verschlussmitteln typischerweise um eine Lasche aus einem ein- oder mehrschichtigen Flachmaterial, welches ausgehend von einer in der Regel um einen distalen Längsrand am freien Ende des betrachteten ersten oder zweiten hinteren Seitenabschnitts nach innen auf den jeweiligen ersten oder zweiten hinteren Seitenabschnitt eingefalteten Konfiguration in eine nach außen ausgefaltete Betriebsstellung entfaltbar ist. Alternativ ist denkbar, dass der erste und/oder zweite hintere Seitenabschnitt innerhalb des frei endenden distalen Abschnitts des ersten und/oder zweiten hinteren Seitenabschnitts jeweils ein funktionales Verschlussmittel ("Patch") aufweist. In an sich bekannter und daher nicht näher zu beschreibender Weise ist ein jeweiliges Verschlussmittel mit klebenden und/oder mechanisch haftenden Bereichen, Schichten oder Elementen ausgestattet, wie zum Beispiel Haken/Schlaufen-Materialien. Sofern der erste und/oder zweite hintere Seitenabschnitt jeweils genau ein Verschlussmittel aufweisen, so erweist es sich als vorteilhaft, wenn dieses Verschlussmittel in der Längsrichtung des jeweiligen ersten und/oder zweiten hinteren Seitenabschnitts ungefähr mittig und im frei endenden distalen Abschnitt des jeweiligen ersten und/oder zweiten hinteren Seitenabschnitts vorgesehen ist. Weiter erweist es sich als vorteilhaft, wenn das betreffende Verschlussmittel eine Erstreckung in der Längsrichtung zwischen 25 % und 75 % einer maximalen Erstreckung C des jeweiligen ersten und/oder zweiten hinteren Seitenabschnitts in der Längsrichtung beträgt. Ferner sind die jeweiligen Verschlussmittel vorzugsweise im ein- und ausgefalteten Zustand rechteckförmig ausgebildet. Sie sind in der herstellerseitigen nicht aktiven Konfiguration vorzugsweise nach innen auf sich selbst eingefaltet.

In der Gebrauchssituation werden der erste und der zweite hintere Seitenabschnitt in Überlappung mit einer körperabgewandten Seite, also einer Außenseite, des Vorderbereichs des Hauptteils gebracht, damit die im Bereich eines jeweiligen in der Windelquerrichtung freien Endes des ersten und zweiten hinteren Seitenabschnitts an beiden hinteren Seitenabschnitten vorgesehenen Verschlussmittel auf die Außenseite des Vorderbereichs des Hauptteils geschlossen werden können. Hierzu sind die Verschlussmittel und mindestens ein Bereich der Außenseite des Vorderbereichs des Hauptteils als Verschlusssystem ausgebildet. Insbesondere weisen die Verschlussmittel hierzu mechanische Komponenten wie Kletthaken auf, insbesondere auch in Kombination mit haftklebenden Bereichen, vermittels derer die Verschlussmittel lösbar haftend mit der Außenseite des Vorderbereichs des Hauptteils in Eingriff bringbar sind. Hierzu hat es sich als vorteilhaft erwiesen, wenn die Außenseite des Vorderbereichs des Hauptteils zumindest bereichsweise, vorzugsweise vollständig durch einen entsprechend ausgebildeten Vliesstoff gebildet ist. Alternativ ist möglich, ein oder mehrere separate Klettflauschelemente auf der Außenseite des Hauptteils im Vorderbereich vorzusehen, welche als Landezone für die Verschlussmittel des ersten und zweiten hinteren Seitenabschnitts dienen.

Weiter bevorzugt weist der Saugkörper der Inkontinenzwegwerfwindel eine maximale Quererstreckung in dem Rückenbereich von QR und eine minimale Quererstreckung in dem Schrittbereich von QS auf, wobei gilt $0{,}9 < QR/QS < 3{,}0$.

Durch eine Taillierung des Saugkörpers kann der Tragekomfort der Inkontinenzwegwerfwindel positiv beeinflusst werden.

Die maximale Quererstreckung des Saugkörpers im Rückenbereich und/oder die minimale Quererstreckung des Saugkörpers im Schrittbereich verlaufen jeweils orthogonal zu der Längsmittelachse LM der Inkontinenzwegwerfwindel und sind in mm zu messen.

Die maximale Quererstreckung des Saugkörpers im Rückenbereich ist an einer in Querrichtung der Inkontinenzwegwerfwindel breitesten Stelle eines Teilbereichs des Saugkörpers zu messen der in einer hinteren Produkthälfte, also zwischen einem hinteren Querrand der Inkontinenzwegwerfwindel und einer Quermittelachse der Inkontinenzwegwerfwindel, platziert ist.

Die minimale Quererstreckung des Saugkörpers im Schrittbereich ist an einer schmalsten Stelle des Saugkörpers im Schrittbereich zu messen. Falls keine schmalste Stelle des Schrittbereichs im Sinne eines echten Minimums vorhanden ist, wie im Falle von zumindest abschnittsweise geraden, parallel zur Längsrichtung verlaufenden Längsrändern des Saugkörpers, dann wird die Messung entlang und auf der Höhe der Quermittelachse der Inkontinenzwegwerfwindel vorgenommen.

Nach einer bevorzugten Ausführungsform weist der erste hintere Seitenabschnitt dieselbe Erstreckung in der Querrichtung auf wie der zweite hintere Seitenabschnitt.

Bevorzugterweise ist der jeweilige in der Querrichtung elastische Bereich des ersten oder zweiten hinteren Seitenabschnitts der Inkontinenzwegwerfwindel durch jeweils eine elastische Komponente mit einer jeweiligen maximalen Erstreckung QE in der Querrichtung gebildet, wobei ein Verhältnis der maximalen Erstreckung QE der elastischen Komponenten des ersten oder zweiten hinteren Seitenabschnitts zu der maximalen Erstreckung C des jeweiligen hinteren Seitenabschnitts der Inkontinenzwegwerfwindel in der Längsrichtung jeweils 0,30-0,70, insbesondere 0,40-0,60, weiter insbesondere 0,45-0,55, weiter insbesondere 0,50-0,43 beträgt.

Der jeweilige elastische Bereich ermöglicht auf vorteilhafte Weise, dass sich die Seitenabschnitte der jeweiligen Körperform und den Bewegungen eines Anwenders besser anpassen und es wird insbesondere der Tragekomfort der Inkontinenzwegwerfwindel erhöht. Der Begriff "Querrichtung" ist hierbei so zu verstehen, dass die Querrichtung in der Gebrauchssituation einer Hüftumfangsrichtung entspricht.

Durch die nur bereichsweise elastische Ausführung des ersten und/oder zweiten hinteren Seitenabschnitts wird erreicht, dass der erste und/oder zweite hintere Seitenabschnitt in der Querrichtung außer einem elastischen Bereich auch mindestens einen, insbesondere zwei nicht elastische und/oder nicht dehnbare Bereiche umfassen, derart, dass vorzugsweise der proximale Abschnitt des Seitenabschnitts zumindest bereichsweise - insbesondere zumindest ein für das Anfügen des ersten und/oder zweiten hinteren Seitenabschnitts an den Hauptteil vorgesehener Teilabschnitt des proximalen Abschnitts - und/oder der frei endende distale Abschnitt zumindest bereichsweise - insbesondere ein für das Anfügen von Verschlussmitteln vorgesehener und/oder an das in Querrichtung der Inkontinenzwegwerfwindel freie Ende des Seitenabschnitts angrenzender Teilabschnitt des frei endenden distalen Abschnitts - nicht elastisch und/oder nicht dehnbar ausgebildet ist. Dabei wird in vorteilhafter Weise das Anfügen des ersten und/oder zweiten hinteren Seitenabschnittes an den Hauptteil und/oder das Anfügen von Verschlussmitteln an den ersten und/oder zweiten hinteren Seitenabschnitt erleichtert.

In einer bevorzugten Ausführungsform beträgt ein Verhältnis einer ersten maximalen Erstreckung QE1 der elastischen Komponente des ersten hinteren Seitenabschnitts zu einer zweiten maximalen Erstreckung QE2 der elastischen Komponente des zweiten hinteren Seitenabschnitts 0,9-1,1, insbesondere 1,0.

Eine einheitliche maximale Erstreckung QE1 und QE2 der jeweiligen elastischen Komponente bei dem ersten und zweiten hinteren Seitenabschnitt erleichtert dem Benutzer die Handhabung der Inkontinenzwegwerfwindeln, da das elastische Verhalten des ersten und zweiten hinteren Seitenabschnitts - wie die maximale Dehnung oder die Rückstellkraft des jeweiligen ersten oder zweiten hinteren Seitenabschnitts beim Aufbringen von Zugkräften während des Fixierens der Windeln am Körper - sich homogen darstellt und eine gleichartige Handhabung ermöglicht.

In einer bevorzugten Ausführungsform weist der elastische Bereich des ersten und zweiten hinteren Seitenabschnitts gleichartige elastische Eigenschaften auf und ist insbesondere aus dem gleichen Material gebildet.

Der Begriff "gleichartige elastische Eigenschaften" ist hierin zu verstehen im Zustand der ersten In-die-Handnahme der Inkontinenzwegwerfwindeln. Sollte die Gleichartigkeit der elastischen Eigenschaften der elastischen Bereiche des ersten und zweiten hinteren Seitenabschnitts nicht bei der ersten In-die-Handnahme ersichtlich sein, so wird mittels der unten beschriebenen Methode zur Prüfung, ob ein Material als "elastisch" einzustufen ist, die bleibende Dehnung (Permanent Set) ermittelt. Im Rahmen der vorliegenden Erfindung werden die elastischen Eigenschaften als gleichartig definiert, wenn der Permanent Set der Materialien gleich groß ist.

Durch die Verwendung von Material mit gleichartigen elastischen Eigenschaften und/oder gleicher maximaler Erstreckung des elastischen Bereichs wird vorteilhaft erreicht, dass sich der erste und der zweite hintere Seitenabschnitt in den durch den Benutzer beim Anlegen der Inkontinenzwegwerfwindel aufzubringenden Zugkräften nicht wesentlich unterscheiden.

Die elastischen Komponenten der Inkontinenzwegwerfwindeln umfassen oder bestehen vorzugsweise aus einem Flachmaterial insbesondere aus einem Vliesstoff, einer Folie, einem textilen Material, einem Schaumstoff oder Kombinationen davon. Insbesondere umfassen oder bestehen die elastischen Komponenten aus einem Laminat aus zwei oder mehreren der genannten Flachmaterialien. Weiter vorzugsweise können die elastischen Komponenten aus einem Verbund von elastischen Materialien und nicht elastischen Materialien gebildet sein, insbesondere aus einem Verbund aus einer elastischen Folie oder einem elastischen Vliesstoff oder einem elastischen Schaumstoff oder elastischen Fäden wie Lycra- oder Spandex- oder Elasthan-Fäden mit einem nicht elastischen und/oder dehnbaren Vliesstoff oder Schaumstoff.

Im Falle eines Verbundes von elastischen Materialien und nicht elastischen Materialien kann der Verbund dadurch als effektive elastische Komponente bereitgestellt werden, dass das nicht elastische Material dehnbar ausgebildet ist und solchen Falls der elastischen Dehnung des elastischen Materials keinen oder nur wenig Widerstand entgegensetzt. Eine weitere Möglichkeit ist, die Materialien im sogenannten, dem Fachmann an sich bekannten stretchbonding Verfahren zu verbinden, mithin das elastische Material in vorgedehntem Zustand mit dem unelastischen Material zu verbinden. Eine weitere Möglichkeit, eine Elastifizierung eines Verbundes von elastischen Materialien und nicht elastischen Materialien zu erreichen, besteht darin, dass der Verbund - vorzugsweise durch eine als "ring rolling" bekannt gewordene Technologie - "aktiviert" wird. Diese Technologie ist beispielsweise in EP 0 650 714 A1 beschrieben. Durch "ring rolling" wird ein an sich undehnbares Verbundmaterial, beispielsweise ein Vlies/Folien-Laminat durch übermäßige Auslenkung zwischen gegeneinander kämmenden Walzen überdehnt. In diesem überdehnten Zustand bringt das zuvor an sich undehnbare Material des Laminates einer Längung im Wesentlichen keinen Widerstand entgegen. Durch Kombination mit einem elastisch dehnbaren Material innerhalb eines derartigen Laminates kann somit eine elastische Dehnbarkeit in dem entsprechend behandelten Bereich erreicht werden.

Zur Prüfung, ob ein Material als "elastisch" in der jeweiligen Richtung einzustufen ist, ist im Rahmen der vorliegenden Erfindung folgende Prüfung der bleibenden Dehnung (Permanent Set) vorzunehmen:
Für die Durchführung der Prüfungen wird eine Zugprüfmaschine Typ Shimadzu AG-Xplus, gemäß DIN EN ISO 7500-01:2004-11, mit unterer fester Klemmbacke und oberer beweglicher Klemmbacke verwendet. Vorzugsweise verfügt die Testvorrichtung über eine Software, welche den Prüfzyklus steuert und ein automatisiertes Aufzeichnen der Kraftwerte sicherstellt.

Bei der Durchführung der Prüfmethode wird der zu prüfende rechteckige Materialabschnitt idealerweise mit einer Breite von 25 mm und einer Länge von 60 mm (die Länge entspricht der Zugrichtung) als Prüfling bereitgestellt.

Hierzu wird der Prüfling zum Beispiel aus einer entsprechenden elastischen Komponente der Inkontinenzwegwerfwindel herausgestanzt, wobei die Zugrichtung des Prüflings der Querrichtung der Inkontinenzwegwerfwindel entspricht.

Idealerweise wird der 25 mm breite und 60 mm lange (Zugrichtung) Prüfling in die Klemmen einer Zugprüfmaschine mit Klemmbackenabstand von 40 mm eingespannt, eine Vorspannung von 0,05 N angefahren und eine zyklische Bewegung (Be- und Entlastung, Prüfgeschwindigkeit 500 mm/min), ohne Haltezeit im oberen Umkehrpunkt, zwischen L0 (Länge des Prüfabschnitts bei Vorspannung von 0,05 N) und einer Maximaldehnung von 60 % durchgeführt. Bei Entlastung wird die Endlänge L1 ebenfalls bei 0,05 N notiert.

Als bleibende Dehnung (permanent set) PS wird berechnet:

$$PS = ((L1-L0):L0)\times100 \ [\%].$$

Als elastisch in der jeweiligen Richtung wird ein Material dann angesehen, wenn die bleibende Dehnung weniger als 25 % beträgt.

Sollte kein 25 mm breiter Abschnitt der zu prüfenden Komponente zur Verfügung stehen, wird ein entsprechend schmalerer Abschnitt verwendet. Sollte kein 60 mm langer Abschnitt der elastischen Komponente zur Verfügung stehen, wird ein entsprechend kürzerer Abschnitt verwendet, wobei der Klemmbackenabstand, mithin die effektive Länge des Prüfabschnitts ggf. auf bis zu 15 mm zu verringern ist. Es ist sicherzustellen, dass der Abschnitt fest in die Klemmbacken eingespannt wird.

Zur Prüfung, ob ein Material oder eine Komponente als "undehnbar" in der jeweiligen Richtung einzustufen ist, ist im Rahmen der vorliegenden Erfindung folgende Prüfung vorzunehmen:

Für die Durchführung der Prüfungen wird eine Zugprüfmaschine Typ Shimadzu AG-Xplus, gemäß DIN EN ISO 7500-01:2004-11, mit unterer fester Klemmbacke und oberer beweglicher Klemmbacke verwendet. Vorzugsweise verfügt die Testvorrichtung über eine Software, welche den Prüfzyklus steuert und ein automatisiertes Aufzeichnen der Kraftwerte sicherstellt.

Bei der Durchführung der Prüfmethode wird der zu prüfende rechteckige Materialabschnitt idealerweise mit einer Breite von 25 mm und einer Länge von 60 mm (die Länge entspricht der Zugrichtung) als Prüfling bereitgestellt. Hierzu wird der Prüfling zum Beispiel aus einer entsprechenden Komponente der Inkontinenzwegwerfwindel herausgestanzt, wobei die Zugrichtung des Prüflings der Querrichtung der Inkontinenzwegwerfwindel entspricht.

Idealerweise wird ein 25 mm breiter und 60 mm langer (Zugrichtung) Prüfling des Materials unter Vorspannung von 0,05 N in die Klemmen der Zugprüfmaschine mit Klemmbackenabstand von 40 mm eingespannt und der Prüfling bis zu einer Kraft von 5 N gedehnt (belastet, Prüfgeschwindigkeit 500 mm/min). Sollte die Dehnung bei der Kraft von 5 N weniger als 30 % betragen, wird das Material im Rahmen der vorliegenden Erfindung als undehnbar bezeichnet. Im Falle, dass der Prüfling bei der Ausführung des vorstehenden Tests bricht, bevor die maximale Kraft von 5 N erreicht ist, wird das Material im Rahmen der vorliegenden Erfindung ebenfalls als "undehnbar" in der jeweiligen Richtung eingestuft.

Sollte kein 25 mm breiter Abschnitt der zu prüfenden Komponente zur Verfügung stehen, wird ein entsprechend schmalerer Abschnitt verwendet. Solchen Falls wird anstelle der Kraft von 5 N der einzustellende Kraftwert errechnet, indem die Belastung von 5 N um den Faktor f1 reduziert wird, welcher sich ergibt aus f1=25/x, wobei x die Breite des Prüflings gemessen in mm ist. Beispiel: ein 12,5 mm breiter Prüfling würde einer Kraft von 2,5 N ausgesetzt werden.

Sollte kein 60 mm langer Abschnitt der elastischen Komponente zur Verfügung stehen, wird ein entsprechend kürzerer Abschnitt verwendet, wobei der Klemmbackenabstand, mithin die effektive Länge des Prüfabschnitts ggf. auf bis zu 15 mm zu verringern ist. Es ist sicherzustellen, dass der Abschnitt fest in die Klemmbacken eingespannt wird.

In einer bevorzugten Variante beträgt ein Verhältnis der jeweiligen maximalen Erstreckung C des ersten hinteren Seitenabschnitts der Inkontinenzwegwerfwindel zu der jeweiligen maximalen Erstreckung C des zweiten hinteren Seitenabschnitts der Inkontinenzwegwerfwindel 0,9-1,1, insbesondere 1,0.

Bevorzugterweise ist die Inkontinenzwegwerfwindel derart ausgebildet, dass im an einen Nutzer angelegten Zustand ein jeweiliger elastischer Bereich zumindest bereichsweise, eine Lücke P zwischen einem jeweiligen ersten oder zweiten vorderen seitlichen Längsrand des Vorderbereichs und einem jeweiligen ersten oder zweiten hinteren seitlichen Längsrand des Rückenbereichs überbrückt.

Der jeweilige elastische Bereich liegt dabei an der Haut des Nutzers an und überlappt insbesondere nicht mit dem Vorderbereich des Hauptteils (Chassismaterial). Das ist insofern vorteilhaft, als der elastische Bereich frei den Bewegungen des Nutzers folgen und wirken kann, ohne am Material des Hauptteils zu reiben, insbesondere ohne das Material des Hauptteils zu raffen, so dass der Tragekomfort erhalten bleibt.

In einem bevorzugten Ausführungsbeispiel umfasst der erste und der zweite hintere Seitenabschnitt jeweils in der Querrichtung einen an den jeweiligen ersten oder zweiten hinteren seitlichen Längsrand anschließenden proximalen Abschnitt und einen an den proximalen Abschnitt anschließenden, das freie Ende des jeweiligen ersten oder zweiten hinteren Seitenabschnitts umfassenden frei endenden distalen Abschnitt,

a) wobei der proximale Abschnitt sich in Querrichtung ausgehend von dem jeweiligen ersten oder zweiten hinteren seitlichen Längsrand über eine Länge erstreckt, die 65% der Erstreckung des jeweiligen ersten oder zweiten hinteren Seitenabschnitts in der Querrichtung beträgt,

b) wobei die in der Querrichtung elastische Komponente des ersten und zweiten hinteren Seitenabschnitts jeweils zumindest bereichsweise, insbesondere vollständig innerhalb des jeweiligen proximalen Abschnitts angeordnet ist,

c) wobei der erste und der zweite hintere Seitenabschnitt jeweils zumindest bereichsweise, insbesondere in dem gesamten jeweiligen distalen Abschnitt in der Querrichtung im Wesentlichen undehnbar ausgebildet sind.

Diese bevorzugte Ausführung bewirkt in vorteilhafter Weise, dass zumindest der frei endende distale Abschnitt, insbesondere zumindest ein Anfassbereich innerhalb des distalen Abschnitts undehnbar ausgebildet und damit für den Anwender sicher greifbar ist.

Das Chassis der Inkontinenzwegwerfwindel im Wesentlichen bildende Materialien, insbesondere das Topsheet, das Backsheet und der Saugkörper sind vorzugsweise undehnbar ausgebildet, um einem Verrutschen der flüssigkeitsabsorbierenden Bereiche, beispielsweise des Saugkörpers, im Tragezustand entgegenzuwirken. Begrenzte Beinöffnungsbereiche des Top- oder Backsheets oder der Auslaufsperren bildenden Cuffelemente können hingegen in an sich bekannter Weise, vorzugsweise durch im stretchbonding Verfahren daran fixierte Elastikfäden elastisch dehnbar ausgebildet sein.

Teilabschnitte des ersten und/oder zweiten Seitenabschnitt insbesondere ein für das Anfügen des jeweiligen ersten oder zweiten hinteren Seitenabschnitts an den Hauptteil vorgesehener Teilabschnitt des proximalen Abschnitts, ein sogenannter Anfügebereich, weiter insbesondere ein für das Anfügen von Verschlussmitteln vorgesehener, sogenannter Verschlussträgerbereich, und/oder an das in Querrichtung der Inkontinenzwegwerfwindel freie Ende des jeweiligen ersten oder zweiten hinteren Seitenabschnitts angrenzender Teilabschnitt des frei endenden distalen Abschnitts, ein sogenannter Anfassbereich, sind bevorzugt aus undehnbarem Material, insbesondere undehnbarem Flachmaterial, ausgebildet, um die Herstellung von stabilen Fügeverbindungen zwischen dem ersten und/oder zweiten hinteren Seitenabschnitt und dem Hauptteil und/oder Verschlussmitteln zu vereinfachen und/oder das Ergreifen des ersten und/oder zweiten hinteren Seitenabschnitts während des Anlegens der Inkontinenzwegwerfwindel durch den Anwender zu erleichtern.

Nach einer bevorzugten Ausführungsform umfassen oder bestehen die genannten undehnbaren Teilabschnitte des ersten und/oder zweiten hinteren Seitenabschnitts, mithin der Anfügebereich und/oder der Verschlussträgerbereich und/oder der Anfassbereich, aus demselben Material, insbesondere aus demselben Flachmaterial, weiter insbesondere aus demselben Vliesstoffmaterial.

Die undehnbaren Flachmaterialien des Chassis und des ersten und/oder zweiten hinteren Seitenabschnitts umfassen oder bestehen vorzugsweise aus einem Vliesstoffmaterial, wie Spinnvlies, Meltblownvlies, Kardenvlies, Spunlacevlies oder Through Air bonded Kardenvlies oder Kombinationen hiervon. Besonders bevorzugt sind Spinnvliesmaterialien (Spunbondvliesstoffe) und/oder Meltblownvliesmaterialien, insbesondere Laminate aus Spunbond- (S) und Meltblown- (M) Vliesstoffen oder Vliesschichten wie SMS-, oder SSMS- oder SMMS-, oder SSMMS- oder SSMMSS-Laminate.

Es hat sich weiter als vorteilhaft erwiesen, wenn das Vliesstoffmaterial zumindest eine Rezepturkomponente auf Basis eines thermoplastischen Polymers enthält, wie Polyethylen PE, Polypropylen PP oder Polyethylenterephthalat PET oder Mischungen davon. Vliesstoffmaterialien umfassend Rayon, Cellulose, Polyamid PA und Mischungen davon sind ebenfalls denkbar.

Das Vliesstoffmaterial hat vorteilhaft ein Flächengewicht von 10 - 70 $g/m^2$, weiter vorzugsweise von 20 - 60 $g/m^2$, weiter vorzugsweise von 30 - 50 $g/m^2$.

Der Saugkörper ist geeignet und dazu bestimmt Körperausscheidungen, insbesondere Körperflüssigkeiten, insbesondere Urin aufzunehmen und dauerhaft zu speichern. Der Saugkörper kann hierzu vorteilhaft superabsorbierendes Polymermaterial (SAP) enthalten, insbesondere zu 5 - 100 Gewichtsprozent, vorzugsweise zu 10 - 95 Gewichtsprozent, weiter vorzugsweise zu 15 - 90 Gewichtsprozent, ganz besonders bevorzugt zu 20 - 80 Gewichtsprozent. Typischerweise kann das SAP-Material zumindest das 15-fache, insbesondere das 20-fache seines Gewichts an 0,9 gewichtsprozentiger Kochsalzlösung absorbieren (gemessen nach NWSP 242.0.R2(15)).

Das SAP-Material kann beispielsweise partikelförmig oder faserförmig oder blatt- oder schaumförmig ausgebildet sein.

Der Saugkörper kann weitere Materialien, wie Zellstofffasern (wood pulp) oder Kunststofffasern enthalten. Denkbar ist weiterhin, den Saugkörper durch Anordnung von ein oder mehreren Schichten verschiedenen Materials, insbesondere aus Vliesstoff auszubilden.

Der Saugkörper ist vorzugsweise integraler Bestandteil des Hauptteils und solchen Falls herstellerseitig unlösbar mit den weiteren Komponenten des Hauptteils verbunden. Solchen Falls ist der Saugkörper vorzugsweise unlösbar mit einem Topsheet und/oder mit einem Backsheet des Hauptteils verbunden.

Der Hauptteil der Inkontinenzwegwerfwindel umfasst vorzugsweise ein zumindest bereichsweise flüssigkeitsdurchlässiges Topsheet, und ein vorzugsweise atmungsaktives und vorzugsweise zumindest bereichsweise flüssigkeitsundurchlässiges Backsheet, die weiter vorzugsweise den Saugkörper sandwichartig umgeben.

Weiterhin kann die Inkontinenzwegwerfwindel auf der körperzugewandten Oberseite des Hauptteils beidseits jeweils eine seitliche Auslaufsperre bildende und/oder beidseits im Wesentlichen entlang der Längsrichtung der Inkontinenzwegwerfwindel verlaufende Cuffelemente aufweisen. Die Cuffelemente umfassen vorzugsweise ein Vliesmaterial ("Cuffvlies"), insbesondere ein hydrophobes Vliesmaterial, oder sind daraus gebildet. Vorzugsweise erstrecken sich Topsheet und/oder Backsheet und/oder Cuffvlies zumindest in Querrichtung der Inkontinenzwegwerfwindel, vorzugsweise auch in Längsrichtung über die Konturränder des Saugkörpers hinaus, um einen entsprechenden Überhang zu bilden. In dem Überhang sind Backsheet und Topsheet und/oder Cuffvlies vorzugsweise zumindest bereichsweise miteinander verbunden, insbesondere durch an sich bekannte Fügeverfahren wie Schweißen, Siegeln, Nähen oder Kleben.

In vorteilhafter Weise weist der Hauptteil in dem Schrittbereich beidseitig an einen jeweiligen Längsrand des Schrittbereichs angrenzend je einen in Längsrichtung der Inkontinenzwegwerfwindel erstreckten elastischen oder elastifizierten Abschnitt, mithin einen elastischen oder elastifizierten Beinöffnungsabschnitt auf. "In Längsrichtung der Inkontinenzwegwerfwindel" bedeutet hierbei, dass der elastische oder elastifizierte Beinöffnungsabschnitt zumindest eine Komponente in Längsrichtung der Inkontinenzwegwerfwindel aufweist, mithin auch schräg oder gekrümmt zur Längsrichtung der Inkontinenzwegwerfwindel verlaufen kann.

Vorzugsweise sind hierzu dem Fachmann an sich bekannte elastische Fäden (Lycra® o.Ä.) in vorgespanntem Zustand an den Hauptteil bildenden Materialien fixiert, vorzugsweise an Top- und/oder Backsheet des Hauptteils, insbesondere in einem Bereich, in dem Top- und/oder Backsheet und/oder Cuffvlies einen Überhang außerhalb der Konturränder des Saugkörpers bilden. Ein elastischer oder elastifizierter Beinöffnungsabschnitt kann nach einer Variante auch durch flachmaterial- oder bandförmige Materialien wie elastische Bänder, Folien, Vliesstoffe oder Schaumstoffe gebildet sein.

An dieser Stelle sei ausgeführt, dass in der vorliegenden Anmeldung jegliche Bemessungsangaben hinsichtlich Quererstreckung, Längserstreckung, Längen und Breiten, Abständen und Spannweiten der Inkontinenzwegwerfwindel oder von jeweiligen Bereichen, Abschnitten oder Komponenten der Inkontinenzwegwerfwindel in eben ausgebreitetem Zustand der die Windel bildenden Flachmaterialien bestimmt werden, so dass die Inkontinenzwegwerfwindel in die in den Figuren dargestellte ebene Konfiguration gebracht werden kann, sofern kein anderer Hinweis auf einen davon abweichenden Zustand angeführt ist. Wenn die Windel beispielsweise durch fadenförmige Elastifizierungsmittel im sogenannten "Stretchbond-Verfahren" elastifiziert wurde, so werden die Flachmaterialien, wie in den Figuren angedeutet, derart ausgedehnt betrachtet, wie sie herstellerseitig als Flachmaterialien zugeführt werden oder nachträglich bis zu ihrer natürlichen Ausgangserstreckung ohne Elastifizierungsmittel ausgebreitet und auf eine ebene Fläche aufgelegt werden können. In dieser ebenen Fläche werden dann die Quererstreckungen, Längserstreckungen oder Abmessungen ermittelt. Dieser Zustand ergibt sich bei undehnbaren Chassismaterialien auf Vliesbasis oder Folienbasis oder Vlies-Folienverbundbasis auf natürliche Weise.

Davon abweichend werden alle Abmessungen der Inkontinenzwegwerfwindel hinsichtlich der Quererstreckung, insoweit sie den in der Querrichtung elastischen Bereich des ersten und/oder zweiten Seitenabschnitts umfassen - namentlich die maximale Spannweite, die erste S1 und zweite Seitenabschnittsbreite S2, der Abstand B, die Spannweite GF1 und die maximale Erstreckung der elastischen Komponente des ersten oder zweiten hinteren Seitenabschnitts - in der Querrichtung lediglich im ausgebreiteten und aufgefalteten aber ungedehnten Zustand vorgenommen. Messungen im ungedehnten Zustand spiegeln den Zustand wider, wie der Anwender das Produkt bei erster In-Die-Handnahme, sowie Entnahme aus der Verpackung, nach Entfaltung der Inkontinenzwegwerfwindel wahrnimmt. Insbesondere werden keine Querzugkräfte auf die Seitenabschnitte, wie sie typischerweise beim Anlegen an den Anwender auftreten, ausgeübt.

Weitere Merkmale, Einzelheiten und Vorteile der Erfindung ergeben sich aus den beigefügten Patentansprüchen und aus der zeichnerischen Darstellung und nachfolgenden Beschreibung bevorzugter Ausführungsformen der Erfindung. In der Zeichnung zeigt:

**Zeichnungen**

**Figur 1**
schematisch, nicht maßstäblich, eine Draufsicht auf eine Inkontinenzwegwerfwindel im aufgefalteten und eben ausgebreiteten Zustand

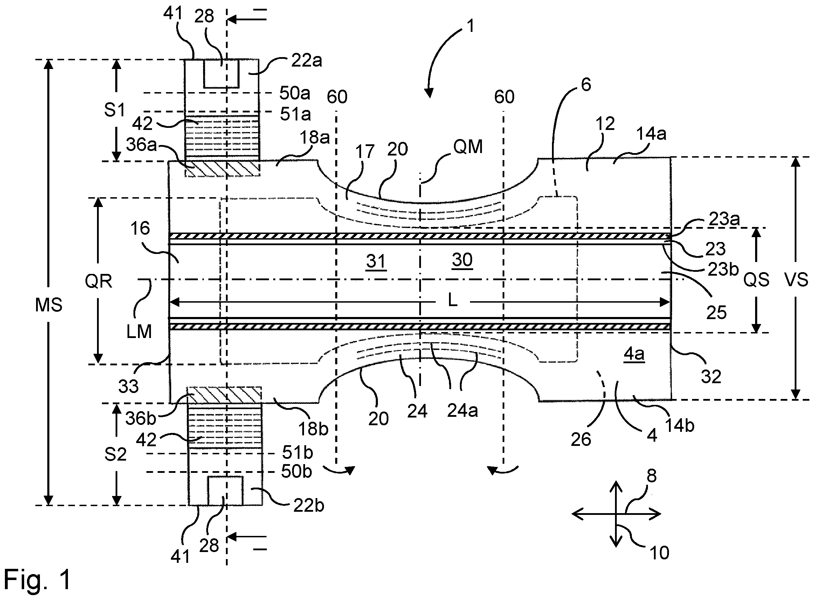

**Figur 2**
schematisch, nicht maßstäblich eine Draufsicht auf die Inkontinenzwegwerfwindel der Figur 1 mit ersten und zweiten Produkt-Falzachsen

**Figur 3a**

schematisch, nicht maßstäblich eine Schnittansicht im Bereich des ersten und zweiten hinteren Seitenabschnitts der Inkontinenzwegwerfwindel der Figur 1

**Figur 3b**

schematisch, nicht maßstäblich eine Schnittansicht einer Inkontinenzwegwerfwindel mit erster Faltanordnung

**Figur 3c**

schematisch, nicht maßstäblich eine Schnittansicht einer Inkontinenzwegwerfwindel mit zweiter Faltanordnung

**Figur 3d**

schematisch, nicht maßstäblich eine Schnittansicht einer Inkontinenzwegwerfwindel mit zweiter und dritter Faltanordnung

**Figur 3e**

schematisch, nicht maßstäblich eine Schnittansicht einer Inkontinenzwegwerfwindel mit zweiter und dritter Faltanordnung und Überlappungsbereich

**Figur 4**

schematisch, nicht maßstäblich, ein Ausschnitt aus einer Inkontinenzwegwerfwindel nach Figur 1 im Bereich eines aufgefalteten, eben ausgebreiteten und ungedehnten ersten hinteren Seitenabschnitts

**Figur 5**

schematisch, nicht maßstäblich, eine Seitenansicht einer Inkontinenzwegwerfwindel im angelegten Zustand

Die Figur 1 zeigt nicht maßstäblich, sondern schematisch eine beispielhafte Inkontinenzwegwerfwindel 1 für die Aufnahme von Körperausscheidungen. Die Inkontinenzwegwerfwindel 1 weist eine Längsrichtung 8, eine Querrichtung 10, eine Längsmittelachse LM, eine Quermittelachse QM, einen Hauptteil 4, einen ersten hinteren Seitenabschnitt 22a und einen zweiten hinteren Seitenabschnitt 22b auf. Der Hauptteil 4 umfasst einen Saugkörper 6, und weist einen Vorderbereich 12 mit einem ersten 14a und einem in Querrichtung 10 gegenüber angeordneten zweiten vorderen seitlichen Längsrand 14b, einen Rückenbereich 16 mit einem ersten 18a und einem in Querrichtung 10 gegenüber angeordneten zweiten hinteren seitlichen Längsrand 18b und einen zwischen dem Vorderbereich 12 und dem Rückenbereich 16 angeordneten Schrittbereich 17.

Der erste hintere Seitenabschnitt 22a ist an den ersten hinteren seitlichen Längsrand 18a angefügt und der zweite hintere Seitenabschnitt 22b ist an den zweiten hinteren seitlichen Längsrand 18b angefügt derart, dass der erste 22a und zweite hintere Seitenabschnitt 22b in der Querrichtung 10 voneinander beabstandet sind, und dass in dem dargestellten aufgefalteten und eben ausgebreiteten Zustand der Inkontinenzwegwerfwindel 1 sich der erste hintere Seitenabschnitt 22a über den ersten hinteren seitlichen Längsrand 18a des Rückenbereichs 16 hinaus und der zweite hintere Seitenabschnitt 22b sich über den zweiten hinteren seitlichen Längsrand 18b des Rückenbereichs 16 hinaus erstreckt.

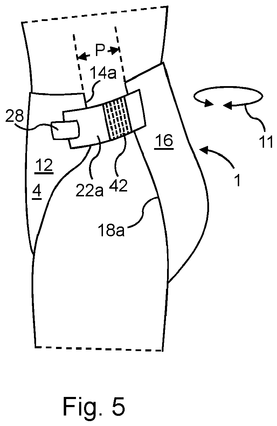

Weiter weisen der erste 22a und der zweite hintere Seitenabschnitt 22b jeweils einen in der Querrichtung 10 elastischen Bereich 42 und an einem jeweiligen freien Ende 41, welches ein nach außen weisendes freies Ende ist, jeweils ein Verschlussmittel 28 auf, so dass der erste 22a und der zweite hintere Seitenabschnitt 22b wie nachfolgend in Figur 5 näher beschrieben zum Anlegen und Schließen der Inkontinenzwegwerfwindel 1 an einen Benutzer jeweils entlang einer Hüftumfangsrichtung 11 um den Körper des Benutzers herumlegbar und in überlappende Anordnung mit einer Außenseite des Vorderbereiches 12 bringbar sind, an der sie über das jeweilige Verschlussmittel 28 jeweils lösbar anhaftbar sind.

Die Hüftumfangsrichtung 11 entspricht im Rückenbereich 16 und im Vorderbereich 12 der Querrichtung 10 der Inkontinenzwegwerfwindel 1.

Optional können der erste 22a und der zweite hintere Seitenabschnitt 22b jeweils mehr als einen in der Querrichtung 10 elastischen Bereich 42 und/oder jeweils mehr als ein Verschlussmittel 28 aufweisen.

Der erste hintere Seitenabschnitt 22a weist eine erste Seitenabschnittsbreite S1 auf und der zweite hintere Seitenabschnitt 22b weist eine zweite Seitenabschnittsbreite S2 auf. Die erste S1 und zweite Seitenabschnittsbreite S2 des jeweiligen ersten 22a oder zweiten hinteren Seitenabschnitts 22b entspricht einer maximalen Erstreckung des jeweiligen ersten 22a oder zweiten hinteren Seitenabschnitts 22b in Querrichtung 10 der Inkontinenzwegwerfwindel 1 über den jeweiligen ersten 18a oder zweiten hinteren seitlichen Längsrand 18b hinaus, gemessen in mm vom jeweiligen ersten 18a oder zweiten hinteren seitlichen Längsrand 18b zu einem jeweiligen nach außen weisenden freien Ende 41 des jeweiligen ersten 22a oder zweiten hinteren Seitenabschnitts 22b im dargestellten aufgefalteten, ungedehnten und eben ausgebreiteten Zustand des ersten 22a und zweiten hinteren Seitenabschnitts 22b.

Die Quermittelachse QM bildet eine imaginäre Grenze zwischen einer vorderen 30 und einer hinteren Produkthälfte 31, wobei die vordere 30 und hintere Produkthälfte 31 in der Längsrichtung 8 gleich weit erstreckt sind. Die vordere Produkthälfte 30 erstreckt sich in der Längsrichtung 8 zwischen einem vorderen Querrand 32 und der Quermittelachse QM der Inkontinenzwegwerfwindel 1. Die hintere Produkthälfte 31 erstreckt sich in der Längsrichtung 8 zwischen einem hinteren Querrand 33 und der Quermittelachse QM der Inkontinenzwegwerfwindel 1.

Der erste 22a und der zweite hintere Seitenabschnitt 22b sind in Figur 1 so positioniert, dass sie von dem hinteren Querrand 33 der Inkontinenzwegwerfwindel 1 in Längsrichtung 8 beabstandet an den Hauptteil 4 angefügt sind, jedoch ist eine Ausführung ohne Abstand, das heißt bündig, in Längsrichtung 8 zwischen dem hinteren Querrand 33 der Inkontinenzwegwerfwindel 1 und dem ersten 22a und/oder zweiten hinteren Seitenabschnitt 22b ebenso denkbar.

Der erste 22a und zweite hintere Seitenabschnitt 22b ist jeweils mit dem Hauptteil 4 überlappend angefügt, wobei der erste 22a und der zweite hintere Seitenabschnitt 22b jeweils innerhalb eines jeweiligen Fügebereiches 36a,36b bevorzugt mittels Ultraschallschweißen mit dem Hauptteil 4 verbunden sind, jedoch ist jedes andere dem Fachmann an sich bekannte und zu dem Zweck geeignete Fügeverfahren, insbesondere Thermoschweißen, Nähen, Siegeln oder Kleben, ebenso möglich.

Wie in Figur 1 nicht maßstäblich, sondern schematisch dargestellt ist die Inkontinenzwegwerfwindel 1 in der Längsrichtung 8 über eine Länge L erstreckt. Die Länge L der Inkontinenzwegwerfwindel 1 ist deren maximale Längserstreckung von dem hinteren Querrand 33 bis zu dem vorderen Querrand 32 der Inkontinenzwegwerfwindel 1.

In Querrichtung 10 ist die Inkontinenzwegwerfwindel 1 über eine maximale Spannweite MS erstreckt. Die maximale Spannweite MS ist eine maximale Quererstreckung der Inkontinenzwegwerfwindel 1 im Rückenbereich 16 der Inkontinenzwegwerfwindel 1, also im Bereich des ersten 22a und zweiten hinteren Seitenabschnitts 22b.

Der Saugkörper 6 der Inkontinenzwegwerfwindel 1 weist eine maximale Quererstreckung QR in dem Rückenbereich 16 und eine minimale Quererstreckung QS in dem Schrittbereich 17 auf. Im in Figur 1 dargestellten nicht maßstäblichen Ausführungsbeispiel weist der Saugkörper 6 eine Taillierung im Schrittbereich 17 auf. Denkbar ist jedoch auch, dass der Saugkörper 6 ohne Wesentliche Taillierung im Schrittbereich 17 ausgebildet ist.

Die maximale Quererstreckung QR des Saugkörpers 6 im Rückenbereich 16 und die minimale Quererstreckung QS des Saugkörpers 6 im Schrittbereich 17 verlaufen jeweils orthogonal zu der nicht maßstäblich, sondern schematisch dargestellten Längsmittelachse LM der Inkontinenzwegwerfwindel 1.

Die maximale Quererstreckung QR des Saugkörpers 6 im Rückenbereich 16 ist an einer in Querrichtung 10 der Inkontinenzwegwerfwindel 1 breitesten Stelle eines Teilbereichs des Saugkörpers 6 innerhalb der hinteren Produkthälfte 31 zu messen, also zwischen dem hinteren Querrand 33 der Inkontinenzwegwerfwindel 1 und der Quermittelachse QM der Inkontinenzwegwerfwindel 1.

Die minimale Quererstreckung QS des Saugkörpers 6 ist an einer schmalsten Stelle des Saugkörpers 6 innerhalb des Schrittbereichs 17 zu messen. Falls keine schmalste Stelle des Schrittbereichs 17 im Sinne eines echten Minimums vorhanden ist, wie im Falle von zumindest abschnittsweise geraden, parallel zur Längsrichtung 8 verlaufenden Längsrändern des Saugkörpers, dann wird die Messung entlang und auf der Höhe der in Figur 1 nicht maßstäblich, sondern schematisch dargestellten Quermittelachse QM der Inkontinenzwegwerfwindel 1 vorgenommen.

Weiter weist die in Figur 1 dargestellte Inkontinenzwegwerfwindel 1 eine vordere Spannweite VS auf, die sich von einem vorderen seitlichen Längsrand 14a in Querrichtung 10 der Inkontinenzwegwerfwindel 1 zum gegenüberliegenden vorderen seitlichen Längsrand 14b erstreckt. Die vordere Spannweite VS ist die maximale Quererstreckung der Inkontinenzwegwerfwindel 1 des Vorderbereichs 12 des Hauptteils 4. In diesem Fall sind im Vorderbereich 12 der Inkontinenzwegwerfwindel 1 keine Seitenabschnitte angefügt. Die vordere Spannweite VS entspricht mithin einer vorderen Hauptteilbreite.

Denkbar ist auch eine Ausführungsform mit an den ersten 14a und/oder zweiten vorderen seitlichen Längsrand 14b des Vorderbereichs 12 beidseits angefügten vorderen Seitenabschnitten. Solchen Falls ist die vordere Spannweite VS von einem in der Querrichtung der Inkontinenzwegwerfwindel freien Ende eines vorderen Seitenabschnitts zum freien Ende eines jeweiligen anderen vorderen Seitenabschnitts erstreckt.

Bevorzugt weist die Inkontinenzwegwerfwindel 1 eine im Vergleich zur maximalen Spannweite MS (im Rückenbereich) signifikant kleinere vordere Spannweite VS auf. Daher wird eine derartige Inkontinenzwegwerfwindel 1 oft auch als T-Form Windel bezeichnet.

In dem in Figur 1 und der unten angefügten Tabelle 1 dargestellten Beispiel weist der Hauptteil 4 im Rückenbereich 16 im Wesentlichen eine gleiche maximale Erstreckung in der Querrichtung 10 wie im Vorderbereich 12 auf. Eine Variante, bei der die in Querrichtung 10 maximale Erstreckung des Hauptteils 4 im Rückenbereich 16 und im Vorderbereich 12 voneinander verschieden sind, ist jedoch ebenso denkbar.

Wie weiter aus Figur 1 ersichtlich umfasst der Hauptteil 4 ein zumindest bereichsweise flüssigkeitsdurchlässiges Topsheet 25, und ein atmungsaktives und zumindest bereichsweise flüssigkeitsundurchlässiges Backsheet 26, die den Saugkörper 6 sandwichartig umgeben. Eine körperzugewandte Oberseite 4a des Hauptteils 4 wird hierbei durch das zentrale, mittig angeordnete zumindest bereichsweise flüssigkeitsdurchlässige Topsheet 25 und das Topsheet 25 beidseits seitlich flankierende Cuffelemente 23 gebildet, welche in an sich bekannter Weise als seitliche Auslaufschutzbarriere dienen und zu diesem Zweck aus einem hydrophoben Vliesmaterial bestehen und in der Längsrichtung 8 elastifiziert sein können. Die Cuffelemente 23 sind zumindest entlang einer Cuffsockellinie 23a an der körperzugewandten Oberseite 4a des Hauptteils 4 festgelegt und weisen einen zumindest abschnittsweise unbefestigten freien Längsrand 23b auf, mit dem sie sich von der körperzugewandten Oberseite 4a des Hauptteils 4 emporerheben können und so die jeweilige seitliche Auslaufsperre bilden.

Der Hauptteil 4 weist weiter in dem Schrittbereich 17 beidseitig an einen jeweiligen Längsrand 20 des Schrittbereichs 17 angrenzend je einen in Längsrichtung 8 der Inkontinenzwegwerfwindel 1 erstreckten elastischen oder elastifizierten Beinöffnungsabschnitt 24 auf. Der elastische oder elastifizierte Beinöffnungsabschnitt 24 weist in diesem Beispiel jeweils zwei elastische Fäden 24a auf, die gekrümmt zur Längsrichtung 8 der Inkontinenzwegwerfwindel 1 verlaufen.

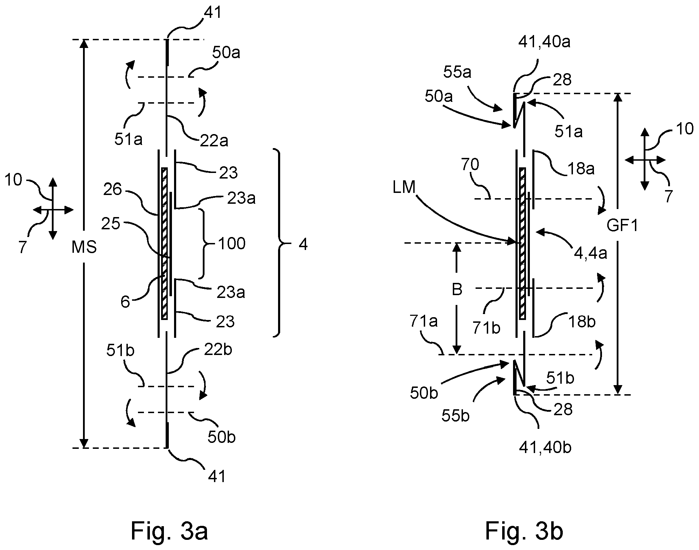

In Figur 1 sind weiterhin beispielhaft eine Anzahl N1=2 an ersten 50a,51a und eine Anzahl N2=2 an zweiten 50b,51b Seitenabschnitt-Falzachsen erkennbar, nämlich eine äußerste erste 50a und eine äußerste zweite Seitenabschnitt-Falzachse 50b und eine jeweils innere erste 51a und innere zweite Seitenabschnitt-Falzachse 51b, an der der jeweilige erste 22a oder zweite hintere Seitenabschnitt 22b wie in Figuren 3a und 3b nachfolgend näher beschrieben bei der Ausbildung einer jeweiligen ersten Faltanordnung des ersten hinteren Seitenabschnitts 55a oder einer ersten Faltanordnung des zweiten hinteren Seitenabschnitts 55b gefaltet werden. Denkbar ist auch, dass der erste 22a und zweite hintere Seitenabschnitt 22b eine andere jeweilige Anzahl N1 an ersten Seitenabschnitt-Falzachsen 50a,51a und N2 an zweiten Seitenabschnitt-Falzachsen 50b,51b aufweist. Weiterhin ist denkbar, dass der erste 22a und der zweite hintere Seitenabschnitt 22b eine unterschiedliche jeweilige Anzahl N1 an ersten Seitenabschnitt-Falzachsen 50a,51a und N2 an zweiten Seitenabschnitt-Falzachsen 50b,51b aufweisen.

Die ersten 50a,51a und die zweiten Seitenabschnitt-Falzachsen 50b,51b verlaufen jeweils entlang der Längsrichtung 8 der Inkontinenzwegwerfwindel 1, insbesondere parallel zur Längsmittelachse LM. Jedoch ist auch eine Ausführungsform denkbar, bei der die ersten 50a,51a und/oder zweiten Seitenabschnitt-Falzachsen 50b,51b nicht streng parallel, insbesondere in einem Winkel $\alpha$ zu der Längsrichtung 8 der Inkontinenzwegwerfwindel 1 verlaufen wobei gilt $0°<\alpha<25°$.

Außerdem sind in Figur 1 schematisch angedeutet zwei Querfalzachsen 60 erkennbar, an welchen die Inkontinenzwegwerfwindel bei der Ausbildung einer vierten Faltanordnung (nicht dargestellt) gefaltet wird. In diesem Beispiel verläuft keine der Querfalzachsen 60 durch den ersten 22a oder zweiten hinteren Seitenabschnitt 22b, insbesondere keine der Querfalzachsen 60 durch die jeweiligen Verschlussmittel 28. Eine andere Anzahl und/oder Anordnung der Querfalzachsen 60, beispielsweise drei oder mehr Querfalzachsen 60, ist ebenso denkbar.

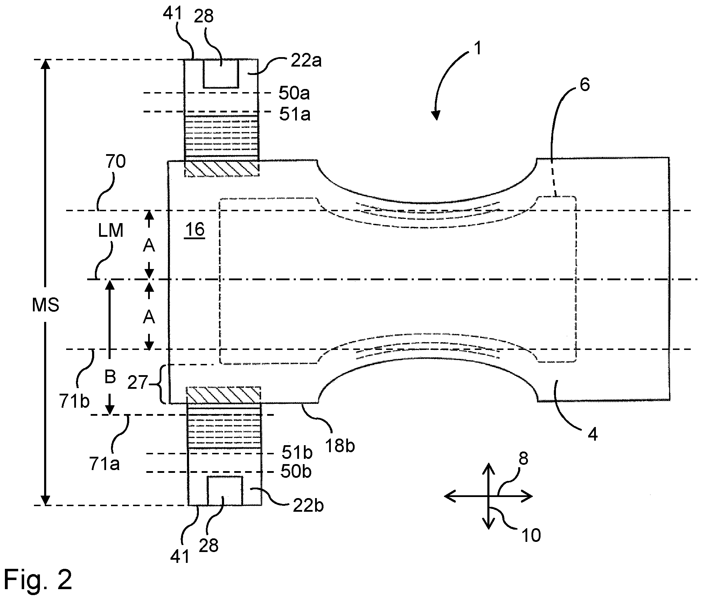

Figur 2 zeigt nicht maßstäblich, sondern schematisch die Inkontinenzwegwerfwindel 1 der Figur 1 mit einer (Anzahl N3=1) von in der Längsrichtung 8 verlaufenden ersten Produkt-Falzachse 70 und zwei (Anzahl N4=2) von in der Längsrichtung 8 verlaufenden zweiten Produkt-Falzachsen 71a,71b. Es gilt also N3<N4.

Denkbar ist, dass die Inkontinenzwegwerfwindel eine andere jeweilige Anzahl N3 oder N4 an ersten 70 und/oder zweiten Produkt-Falzachsen 71a,71b aufweist, solange gilt N3<N4.

Die erste 70 und die zweiten Produkt-Falzachsen 71a,71b verlaufen in diesem Ausführungsbeispiel jeweils parallel zur Längsmittelachse LM der Inkontinenzwegwerfwindel 1. Jedoch ist auch eine Ausführungsform denkbar, bei der die ersten 70 und/oder zweiten Produkt-Falzachsen 71a,71b nicht streng parallel, insbesondere in einem Winkel $\beta$ zu der Längsrichtung 8 der Inkontinenzwegwerfwindel 1 verlaufen wobei gilt $0°<\beta<25°$.

Eine jeweilige in der Querrichtung 10 innerste in der Längsrichtung 8 verlaufende erste Produktfalzachse 70 ist die der Längsmittelachse LM jeweils nächstgelegene erste Produktfalzachse 70, und eine jeweilige in der Querrichtung 10 innerste in der Längsrichtung 8 verlaufende zweite Produktfalzachse 71b ist die der Längsmittelachse LM jeweils nächstgelegene erste Produktfalzachse 71b. Bei der beispielhaften Inkontinenzwegwerfwindel 1 der nicht maßstäblichen sondern schematischen Figur 2 sind die innerste erste 70 und innerste zweite Produktfalzachse 71b in einem gleichen Abstand A zur Längsmittelachse LM angeordnet, hierbei sind also die innerste erste 70 und innerste zweite Produktfalzachse 71b spiegelsymmetrisch zueinander mit der Längsmittelachse LM als Symmetrieachse angeordnet.

Die Inkontinenzwegwerfwindel 1 weist eine in der Querrichtung 10 äußerste zweite Produkt-Falzachse 71a auf. Die äußerste zweite Produkt-Falzachse 71a ist wie in Figur 2 leicht erkennbar diejenige der zweiten Produkt-Falzachsen 71a,71b, die im aufgefalteten und eben ausgebreiteten Zustand der Inkontinenzwegwerfwindel den größten Abstand B in Querrichtung zu der Längsmittelachse LM der Inkontinenzwegwerfwindel aufweist.

Die in der Querrichtung 10 äußerste in der Längsrichtung 8 verlaufende zweite Produktfalzachse 71a verläuft in diesem Beispiel innerhalb des zweiten hinteren Seitenabschnitts 22b und außerhalb des Hauptteils 4 und damit auch außerhalb des Saugkörpers 6.

Alternativ kann die in der Querrichtung 10 äußerste in der Längsrichtung 8 verlaufende zweite Produktfalzachse 71a entlang des zweiten hinteren seitlichen Längsrandes 18b des Rückenbereiches 16 verlaufen.

Als weitere Alternative kann die in der Querrichtung 10 äußerste in der Längsrichtung 8 verlaufende zweite Produktfalzachse 71a innerhalb des Hauptteils 4 und außerhalb des Saugkörpers 6, insbesondere in Querrichtung 10 zwischen dem Saugkörper 6 und dem zweiten hinteren seitlichen Längsrand 18b des Rückenbereichs 16, weiter insbesondere im Bereich eines von den in Figur 1 näher beschriebene Backsheet 26 und dem Topsheet 25 und/oder dem Cuffelement 23 ausgebildeten und sich in Querrichtung 10 über den Saugkörper 6 hinaus erstreckenden seitlichen Überhanges 27 verlaufen, so, dass der Saugkörper 6 nicht die in der Längsrichtung 8 verlaufende zweite Produktfalzachse 71a überfängt.

Weitere in der Figur 2 erkennbare Bezugszeichen sind mit Bezug zur Figur 1 näher beschrieben. Gleiche Bezugszeichen bezeichnen dabei die gleichen Bauteile oder Seitenabschnitt-Falzachsen oder sonstige Details der Inkontinenzwegwerfwindel.

Die Figuren 3a bis 3e zeigen jeweils eine Schnittansicht durch den Rückenbereich der Inkontinenzwegwerfwindel 1 im Bereich des ersten 22a und zweiten hinteren Seitenabschnitts 22b. Die Schnittansicht ist entlang einer Ebene in der Querrichtung 10 und in einer zur Querrichtung 10 und zu der nicht gezeigten Längsrichtung 8 verlaufenden Dickenrichtung 7.

Figuren 3a zeigt eine Schnittansicht entlang der Ebene I-I der Figur 1. Erkennbar ist die maximale Spannweite MS, also die maximale Erstreckung der Inkontinenzwegwerfwindel 1 in Querrichtung 10 im aufgefalteten und eben ausgebreiteten Zustand. Angedeutet sind weiterhin eine Anzahl N1=2 erste 50a,51a und eine Anzahl N2=2 zweite Seitenabschnitt-Falzachsen 50b,51b, dabei also N1=N2, an welchen bei der Ausbildung der nachfolgend in Figur 3b näher beschriebenen jeweiligen ersten Faltanordnung 55a,55b der jeweilige erste 22a und zweite hintere Seitenabschnitt 22b, vorzugsweise z-förmig, auf sich selbst gefaltet wird. Die ersten 50a,51a und zweiten 50b,51b Seitenabschnitt-Falzachsen verlaufen innerhalb des jeweiligen ersten 22a oder zweiten hinteren Seitenabschnitts 22b in der Längsrichtung 8 der Inkontinenzwegwerfwindel 1.

Der Hauptteil 4 umfasst das zumindest bereichsweise flüssigkeitsdurchlässige Topsheet 25, beidseits zumindest abschnittsweise eine seitliche Auslaufsperre bildende und beidseits entlang der Längsrichtung 8 verlaufende Cuffelemente 23, und ein vorzugsweise atmungsaktives und vorzugsweise zumindest bereichsweise flüssigkeitsundurchlässiges Backsheet 26, die den Saugkörper 6 sandwichartig umgeben. Zwischen den inneren Längsrändern 23a der Cuffelemente 23 ist ein Cuffelemente-freier Bereich 100 erkennbar. Der erste 22a und der zweite hintere Seitenabschnitt 22b weisen jeweils ein nach außen weisendes freies Ende 41 auf.

Figur 3b zeigt schematisch, nicht maßstäblich, in einer der Figur 3a analogen Schnittansicht der Inkontinenzwegwerfwindel 1 den ersten 22a und zweiten hinteren Seitenabschnitt 22b mit einer Z-förmigen (mit (N1,N2)=2) ersten Faltanordnung des ersten hinteren Seitenabschnitts 55a und einer Z-förmigen (mit (N1,N2)=2) ersten Faltanordnung des zweiten hinteren Seitenabschnitts 55b.

Wie oben ausgeführt kann die erste Faltanordnung des ersten hinteren Seitenabschnitts 55a und/oder des zweiten hinteren Seitenabschnitts 55b für die jeweilige Anzahl (N1,N2) einen Wert von 1-5 aufweisen, insbesondere anstatt (N1, N2)=2 auch (N1,N2)=3 oder (N1,N2)=1 erste 50a,51a und/oder zweite Seitenabschnitt-Falzachsen 50b,51b aufweisen. Solchen Falls sind der erste 22a und/oder zweite hintere Seitenabschnitt 22b insbesondere M- oder W-förmig (bei (N1,N2)=3) oder C-förmig (bei (N1,N2)=1) zur ersten Faltanordnung gefaltet.

Erkennbar ist eine Spannweite GF1 der Inkontinenzwegwerfwindel 1 mit erster Faltanordnung 55a,55b. Die Spannweite GF1 der Inkontinenzwegwerfwindel 1 mit erster Faltanordnung 55a,55b ist die maximale Erstreckung der Inkontinenzwegwerfwindel 1 in Querrichtung 10 im Bereich der auf sich selbst gefalteten ersten 22a und zweiten hinteren Seitenabschnitte 22b, also von einem äußeren Ende eines auf sich selbst gefalteten ersten hinteren Seitenabschnittes 40a zu dem äußeren Ende eines auf sich selbst gefalteten zweiten hinteren Seitenabschnittes 40b gemessen in mm. In diesem Ausführungsbeispiel entspricht das äußere Ende des auf sich selbst gefalteten ersten hinteren Seitenabschnitts 40a dem freien Ende 41 des ersten hinteren Seitenabschnitts 22a. Das äußere Ende des auf sich selbst gefalteten zweiten hinteren Seitenabschnitts 40b entspricht hier dem freien Ende 41 des zweiten hinteren Seitenabschnitts 22b. Andere Anordnungen des freien Endes 41 des ersten oder zweiten hinteren Seitenabschnitts und eines jeweiligen äußeren Endes eines auf sich selbst gefalteten ersten oder zweiten hinteren Seitenabschnitts, insbesondere bei M-oder C-förmiger erster Faltanordnung des ersten 55a oder zweiten hinteren Seitenabschnitts 55b sind ebenso möglich und denkbar.

Vorzugsweise (nicht dargestellt) ist die erste Faltanordnung lösbar fixiert, indem die - im dargestellten Fall z-förmig - übereinander liegenden Teilabschnitte der Faltanordnung durch Haftvermittler wie Klebstoff oder durch wenige Schweißpunkte, insbesondere Ultraschallschweißpunkte vorläufig aneinandergefügt sind.

Weiter zeigt Figur 3b schematisch angedeutet die Position der Längsmittelachse LM, die erste Produktfalzachse 70, die zweiten Produktfalzachsen 71a,71b und den Abstand B der äußersten zweiten Produktfalzachse 71a von der Längsmittelachse LM.

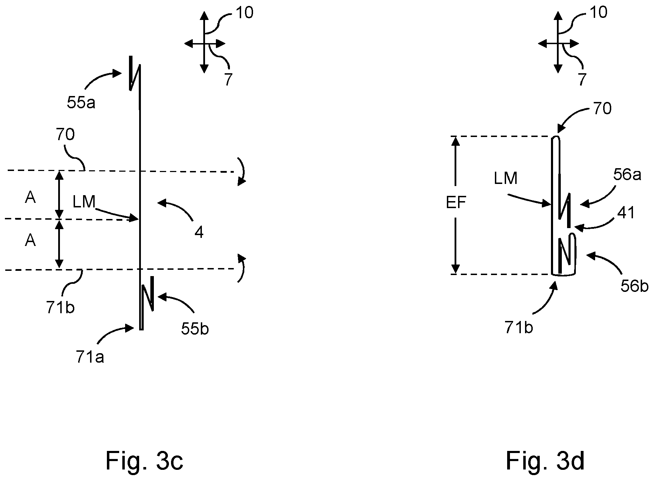

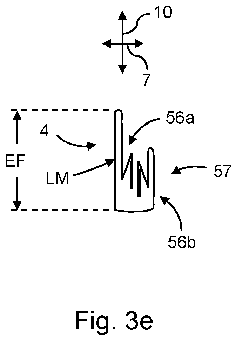

Bei der Ausbildung der in Figur 3c,3d,3e näher beschriebenen zweiten 56a und dritten Faltanordnung 56b wird die erste Faltanordnung des ersten hinteren Seitenabschnitts 55a an einer Anzahl N3 der in der Längsrichtung 8 verlaufenden ersten Produktfalzachsen 70 nach innen auf eine körperzugewandte Oberseite 4a des Hauptteils zu einer zweiten Faltanordnung 56a gefaltet und die erste Faltanordnung des zweiten hinteren Seitenabschnitts 55b wird an einer Anzahl N4 der in der Längsrichtung 8 verlaufenden zweiten Produktfalzachsen 71a,71b nach innen auf die körperzugewandte Oberseite 4a des Hauptteils 4 zu einer dritten Faltanordnung 56b gefaltet.

In der in Figur 3b dargestellten Schnittansicht ist erkennbar, dass die in der Querrichtung 10 äußerste in der Längsrichtung 8 verlaufende zweite Produktfalzachse 71a innerhalb des zweiten hinteren Seitenabschnitts 22b und außerhalb der ersten Faltanordnung 55b des zweiten hinteren Seitenabschnitts 22b verläuft.

In Figur 3b ist außerdem zu erkennen, dass das jeweilige Verschlussmittel 28 des ersten 22a und zweiten hinteren Seitenabschnitts 22b in der ersten Faltanordnung 55a,55b außerhalb des jeweiligen ersten 18a oder zweiten hinteren seitlichen Längsrandes 18b des Rückenbereichs 16, das heißt außerhalb des Hauptteils 4, zu liegen kommt.

Figur 3c zeigt in einer den Figuren 3a und 3b analogen Schnittansicht die Konfiguration nach Einschlagen der ersten Faltanordnung des zweiten hinteren Seitenabschnitts 55b nach innen auf die körperzugewandte Oberseite 4a des Hauptteils 4 der Inkontinenzwegwerfwindel 1 entlang der äußersten zweiten Produktfalzachse 71a. Erkennbar sind die innerste zweite Produktfalzachse 71b, an der der zweite hintere Seitenabschnitt 22b bei der Ausbildung der dritten Faltanordnung 56b weiter auf die körperzugewandte Oberseite 4a des Hauptteils 4 eingeschlagen wird, und die erste Produktfalzachse 70, an der die erste Faltanordnung des ersten hinteren Seitenabschnitts 55a bei der Ausbildung der zweiten Faltanordnung 56a auf die körperzugewandte Oberseite 4a des Hauptteils 4 eingeschlagen wird. Schematisch angedeutet sind außerdem die Position der Längsmittelachse LM und der Abstand A den die erste Produktfalzachse 70 und die innerste zweite Produktfalzachse 71b jeweils zur Längsmittelachse LM in Querrichtung 10 aufweisen.

Für Zwecke der Übersichtlichkeit sind in der Ansicht der Figur 3c und in nachfolgenden Figuren 3d und 3e die Flachmaterialien des Hauptteils (Topsheet, Cuffelemente, Saugkörper, Backsheet) zusammengefasst, zeichnerisch nicht voneinander getrennt und nicht unterscheidbar als eine einzige Schicht illustriert.

Figur 3d zeigt in einer den Figuren 3a,3b und 3c analogen Schnittansicht die Konfiguration nach Einschlagen der ersten Faltanordnung des ersten hinteren Seitenabschnitts 55a nach innen auf die körperzugewandte Oberseite 4a des Hauptteils 4 der Inkontinenzwegwerfwindel 1 entlang der ersten Produktfalzachse 70 (zweite Faltanordnung 56a), und nach einem vollständigen Einschlagen der ersten Faltanordnung des zweiten hinteren Seitenabschnitts 55b nach innen auf die körperzugewandte Oberseite 4a des Hauptteils 4 der Inkontinenzwegwerfwindel 1 entlang der zweiten Produktfalzachsen 71a,71b (dritte Faltanordnung 56b).

Hierbei kommen die beiden gefalteten Seitenabschnitte in Querrichtung 10 nicht übereinander, sondern nebeneinander zu liegen. In diesem Beispiel sind der erste 22a und der zweite hintere Seitenabschnitt 22b in der Querrichtung voneinander beabstandet. Denkbar ist auch, dass der erste 22a und der zweite hintere Seitenabschnitt 22b auf Stoß zu liegen kommen.

Schematisch angedeutet ist die Position der Längsmittelachse LM. Dadurch ist erkennbar, dass die zweite Faltanordnung 56a die Längsmittelachse LM zumindest bereichsweise überfängt.

Erkennbar ist, dass der erste hintere Seitenabschnitt 22a an dem freien Ende 41 ergriffen werden kann, wodurch die zweite Faltanordnung in einem Zug öffenbar, das heißt auffaltbar gestaltet werden kann.

Figur 3d zeigt außerdem die Endfaltbreite EF der Inkontinenzwegwerfwindel 1 mit zweiter 56a und dritter Faltanordnung 56b.

Figur 3e zeigt in einer den Figuren 3a,3b,3c und 3d analogen Schnittansicht eine Alternative zu der in Figur 3d dargestellten Konfiguration nach Ausbildung der zweiten 56a und dritten 56b Faltanordnung. Hierbei kommen die beiden gefalteten Seitenabschnitte in der dargestellten Ausführungsform in der Dickenrichtung 7 der Inkontinenzwegwerfwindel 1 bereichsweise übereinander zu liegen, die zweite 56a und die dritte Faltanordnung 56b überlappen also zumindest bereichsweise zur Bildung eines Überlappungsbereiches 57 miteinander. Die zweite Faltanordnung 56a ist in dem Überlappungsbereich 57 zwischen der dritten Faltanordnung 56b und einer körperzugewandten Oberseite 4a des Hauptteils 4 angeordnet.

Figur 3e zeigt außerdem schematisch die Position die Längsmittelachse LM und die Endfaltbreite EF der Inkontinenzwegwerfwindel 1 mit zweiter 56a und dritter Faltanordnung 56b.

Es ist erkennbar, dass in der überlappenden Konfiguration der zweiten 56a und dritten Faltanordnung 56b die zweite Faltanordnung 56a die Längsmittelachse LM überfängt, insbesondere ist der von der zweiten 56a und dritten Faltanordnung 56b ausgebildete Überlappungsbereich 57 vollständig zwischen den hier nicht dargestellten Cuffelementen, mithin in dem Cuffelement-freien Bereich angeordnet.

Vorzugsweise wird die Inkontinenzwegwerfwindel mit zweiter 56a und dritter Faltanordnung 56b anschließend zum Zwecke der Verpackung entlang der in Figur 1 angedeuteten Querfalzachsen 60 nach innen gefaltet.

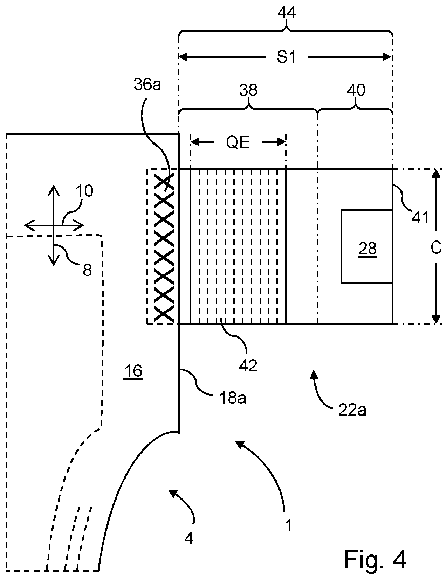

Figur 4 zeigt nicht maßstäblich, sondern schematisch einen Ausschnitt der Inkontinenzwegwerfwindel 1 nach Figur 1 in vergrößerter Darstellung, und zwar im Bereich des ersten hinteren Seitenabschnitts 22a in aufgefaltetem und eben ausgebreitetem, jedoch nicht gedehntem Zustand. Der erste hintere Seitenabschnitt 22a ist im Rückenbereich 16 der Inkontinenzwegwerfwindel 1 an den ersten hinteren seitlichen Längsrand 18a des Hauptteils 4 angefügt und weist vorzugsweise im Bereich seines in der Querrichtung 10 freien Endes 41 zumindest ein Verschlussmittel 28, insbesondere genau ein Verschlussmittel 28 auf. Der erste hintere Seitenabschnitt 22a ist mit dem Hauptteil 4 überlappend angefügt,

EP 3 858 310 B1

wobei der erste hintere Seitenabschnitt 22a innerhalb eines Fügebereiches 36a mit dem Hauptteil 4 verbunden ist.

Weiter weist der erste hintere Seitenabschnitt 22a einen in der Querrichtung 10 elastischen Bereich 42 auf, der durch vorzugsweise eine elastische Komponente mit einer maximalen Erstreckung QE in der Querrichtung 10 gebildet ist. Der erste hintere Seitenabschnitt 22a weist außerdem in Längsrichtung 8 eine maximale Erstreckung C auf.

Die Darstellung in Figur 4 ist insbesondere so zu verstehen, dass ein nicht gezeigter zweiter hinterer Seitenabschnitt 22b, der an einen in Querrichtung 10 gegenüberliegenden zweiten hinteren seitlichen Längsrand 18b des Hauptteils 4 angefügt ist, im Wesentlichen identisch, dabei spiegelsymmetrisch zu dem dargestellten ersten hinteren Seitenabschnitt 22a ausgebildet ist.

In der Querrichtung 10 umfasst der erste hintere Seitenabschnitt 22a einen an den ersten hinteren seitlichen Längsrand 18a anschließenden proximalen Abschnitt 38 und einen an den proximalen Abschnitt 38 anschließenden, frei endenden distalen Abschnitt 40. Der proximale Abschnitt 38 erstreckt sich in Querrichtung 10 ausgehend von dem ersten hinteren seitlichen Längsrand 18a über eine Länge, die 65% der Erstreckung 44 des ersten hinteren Seitenabschnitts 22a in der Querrichtung 10 beträgt. Die in der Querrichtung 10 elastische Komponente des ersten hinteren Seitenabschnitts 22a ist zumindest bereichsweise, insbesondere vollständig innerhalb des proximalen Abschnitts 38 angeordnet.

Die Erstreckung 44 des ersten hinteren Seitenabschnitts 22a in der Querrichtung 10 entspricht der ersten Seitenabschnittsbreite S1 des ersten hinteren Seitenabschnitts 22a und ist zu verstehen als maximale Erstreckung des ersten hinteren Seitenabschnitts 22a in Querrichtung 10 der Inkontinenzwegwerfwindel 1 über den ersten hinteren seitlichen Längsrand 18a hinaus, gemessen in mm vom ersten hinteren seitlichen Längsrand 18a zum nach außen weisenden freien Ende 41 des ersten hinteren Seitenabschnitts 22a im aufgefalteten, ungedehnten und eben ausgebreiteten Zustand.

Der erste hintere Seitenabschnitt 22a ist zumindest bereichsweise, insbesondere in dem gesamten distalen Abschnitt 40 in der Querrichtung 10 im Wesentlichen undehnbar ausgebildet.

In Figur 5 ist nicht maßstäblich sondern schematisch eine Seitenansicht der Inkontinenzwegwerfwindel 1 im an einen Benutzer angelegten Zustand gezeigt. Der erste 22a und der nicht gezeigte zweite hintere Seitenabschnitt 22b werden zum Anlegen und Schließen der Inkontinenzwegwerfwindel 1 an den Benutzer jeweils entlang einer Hüftumfangsrichtung 11 um den Körper des Benutzers herumgelegt und in überlappende Anordnung mit einer Außenseite des Vorderbereiches 12 gebracht, an der sie über das jeweilige Verschlussmittel 28 jeweils lösbar anhaftbar sind.

Die Inkontinenzwegwerfwindel 1 ist vorzugsweise derart ausgebildet, dass im an einen Benutzer angelegten Zustand der jeweilige elastische Bereich 42 des ersten 22a und zweiten hinteren Seitenabschnitts 22b zumindest bereichsweise, eine Lücke P zwischen dem jeweiligen vorderen seitlichen Längsrand 14a,14b, also einem Seitenrand des Vorderbereichs 12 und einem jeweiligen ersten 18a oder zweiten hinteren seitlichen Längsrand 18b, also einem Seitenrand des Rückenbereichs 16 überbrückt. Dabei liegt der jeweilige elastische Bereich 42 vorzugsweise an der Haut des Benutzers an und wird insbesondere nicht durch ein Material des Vorderbereichs 12 des Hauptteils 4 abgedeckt.

Beispiel

In nachfolgender Tabelle 1 sind Eigenschaften eines Ausführungsbeispiels einer bevorzugten Inkontinenzwegwerfwindel dargestellt. Der erste und der zweite hintere Seitenabschnitt sind (im ausgefalteten Zustand) jeweils im Wesentlichen gleich, mithin spiegelsymmetrisch ausgebildet.

Die in Tabelle 1 bezeichneten Parameter MS, GF1, EF sind in Figuren 3a bis 3e veranschaulicht.

Tabelle 1: Abmessungen einer beispielhaften Inkontinenzwegwerfwindel

| Parameter | Wert |
|---|---|
| Länge L | 810 mm |
| Maximale Spannweite MS | 790 mm |
| Vordere Spannweite VS | 330 mm |
| Vordere Hauptteilbreite | 330 mm |
| Erste Seitenabschnittsbreite S1 | 230 mm |
| Zweite Seitenabschnittsbreite S2 | 230 mm |
| Abstand A | 95 mm |
| Abstand B | 185 mm |
| GF1 | 510 mm |

18

(fortgesetzt)

| Parameter | Wert |
|---|---|
| Endfaltbreite EF | 190 mm |
| QR | 245 mm |
| QS | 170 mm |
| Verhältnis QR/QS | 1,44 |
| Verhältnis MS zu VS (MS/VS) | 2,4 |
| Verhältnis GF1 zu MS (GF1/MS) | 0,65 |
| Verhältnis EF zu MS (EF/MS) | 0,24 |
| Maximale Erstreckung C des ersten hinteren Seitenabschnitts | 140 mm |
| Maximale Erstreckung C des zweiten hinteren Seitenabschnitts | 140 mm |
| Maximale Erstreckung QE | 70 mm |
| Verhältnis der maximalen Erstreckung C des ersten zu der maximalen Erstreckung C des zweiten hinteren Seitenabschnitts | 1,0 |
| Verhältnis QE zu C (QE/C) | 0,50 |
| Anzahl N1 an ersten Seitenabschnitt-Falzachsen | 2 |
| Anzahl N2 an zweiten Seitenabschnitt-Falzachsen | 2 |
| Anzahl N3 an ersten Produktfalzachsen | 1 |
| Anzahl N4 an zweiten Produktfalzachsen | 2 |
| Anzahl an Querfalzachsen | 2 |

**Patentansprüche**

1. Inkontinenzwegwerfwindel mit einer Längsrichtung (8), einer Querrichtung (10), einer Längsmittelachse LM, und einem Hauptteil (4) umfassend einen Saugkörper (6), wobei der Hauptteil (4) einen Vorderbereich (12) mit einem ersten (14a) und einem in Querrichtung (10) gegenüber angeordneten zweiten vorderen seitlichen Längsrand (14b) und einen Rückenbereich (16) mit einem ersten (18a) und einem in Querrichtung (10) gegenüber angeordneten zweiten hinteren seitlichen Längsrand (18b) aufweist, und wobei der Hauptteil (4) einen zwischen dem Vorderbereich (12) und dem Rückenbereich (16) angeordneten Schrittbereich (17) aufweist, und wobei der Hauptteil (4) einen ersten hinteren Seitenabschnitt (22a) und einen zweiten hinteren Seitenabschnitt (22b) aufweist, wobei der erste hintere Seitenabschnitt (22a) an den ersten hinteren seitlichen Längsrand (18a) angefügt ist und wobei der zweite hintere Seitenabschnitt (22b) an den zweiten hinteren seitlichen Längsrand (18b) angefügt ist, wobei der erste (22a) und der zweite hintere Seitenabschnitt (22b) jeweils einen in der Querrichtung (10) elastischen Bereich (42) aufweisen, wobei der erste (22a) und der zweite hintere Seitenabschnitt (22b) jeweils wenigstens ein Verschlussmittel (28) aufweisen, so dass der erste (22a) und der zweite hintere Seitenabschnitt (22b) zum Anlegen und Schließen der Inkontinenzwegwerfwindel an einen Benutzer jeweils entlang einer Hüftumfangsrichtung um den Körper des Benutzers herumlegbar und in überlappende Anordnung mit einer Außenseite des Vorderbereiches (12) bringbar sind, an der sie über das jeweilige Verschlussmittel (28) jeweils lösbar anhaftbar sind,

    a) wobei der erste hintere Seitenabschnitt (22a) an einer Anzahl N1 in der Längsrichtung (8) verlaufender erster Seitenabschnitt-Falzachsen (50a, 51a) zu einer ersten Faltanordnung des ersten hinteren Seitenabschnitts (55a) auf sich selbst gefaltet ist,
    b) wobei der zweite hintere Seitenabschnitt (22b) an einer Anzahl N2 in der Längsrichtung (8) verlaufender zweiter Seitenabschnitt-Falzachsen (50b, 51b) zu einer ersten Faltanordnung des zweiten hinteren Seitenabschnitts (55b) auf sich selbst gefaltet ist,
    c) wobei die erste Faltanordnung des ersten hinteren Seitenabschnitts (55a) an einer Anzahl N3 von in der Längsrichtung (8) verlaufenden ersten Produktfalzachsen (70) nach innen auf eine körperzugewandte Oberseite (4a) des Hauptteils (4) gefaltet ist zu einer zweiten Faltanordnung (56a),

d) wobei die erste Faltanordnung (55b) des zweiten hinteren Seitenabschnitts (22b) an einer Anzahl N4 von in der Längsrichtung (8) verlaufenden zweiten Produktfalzachsen (71a,71b) nach innen auf die körperzugewandte Oberseite (4a) des Hauptteils (4) gefaltet ist zu einer dritten Faltanordnung (56b),

e) wobei gilt N3<N4.

2. Inkontinenzwegwerfwindel nach Anspruch 1 **dadurch gekennzeichnet, dass** gilt N3≥1, N4≤4, insbesondere N3=1 und N4≥2, wobei insbesondere gilt N4=2.

3. Inkontinenzwegwerfwindel nach mindestens einem der vorgenannten Ansprüche, **dadurch gekennzeichnet, dass** die Anzahl N1 der in Längsrichtung (8) verlaufenden ersten Seitenabschnitt-Falzachsen (50a,51a) und die Anzahl N2 der in Längsrichtung (8) verlaufenden zweiten Seitenabschnitt-Falzachsen (50b,51b) jeweils 1-5, insbesondere 1-4, weiter insbesondere 1-3 beträgt.

4. Inkontinenzwegwerfwindel nach mindestens einem der vorgenannten Ansprüche, wobei gilt N1=N2, wobei insbesondere gilt N1=2, wobei weiter insbesondere die erste Faltanordnung des ersten hinteren Seitenabschnitts (55a) und die erste Faltanordnung des zweiten hinteren Seitenabschnitts (55b) jeweils Z-förmig ausgebildet ist.

5. Inkontinenzwegwerfwindel nach mindestens einem der vorgenannten Ansprüche, wobei das wenigstens eine Verschlussmittel (28) des ersten (22a) und/oder zweiten hinteren Seitenabschnitts (22b) an einem freien Ende (41) des jeweiligen ersten (22a) oder zweiten hinteren Seitenabschnitts (22b) angeordnet ist.

6. Inkontinenzwegwerfwindel nach mindestens einem der vorgenannten Ansprüche, wobei das wenigstens eine Verschlussmittel (28) des ersten (22a) und/oder zweiten hinteren Seitenabschnitts (22b) in der ersten Faltanordnung (55a,55b) außerhalb des jeweiligen ersten (18a) oder zweiten hinteren seitlichen Längsrandes (18b) des Rückenbereichs (16), das heißt außerhalb des Hauptteils (4), zu liegen kommt.

7. Inkontinenzwegwerfwindel nach mindestens einem der vorgenannten Ansprüche, wobei eine in der Querrichtung (10) äußerste in der Längsrichtung (8) verlaufende zweite Produktfalzachse (70) außerhalb des Saugkörpers (6), insbesondere innerhalb des zweiten hinteren Seitenabschnitts (22b), weiter insbesondere innerhalb des zweiten hinteren Seitenabschnitts (22b) und außerhalb der ersten Faltanordnung (55b) des zweiten hinteren Seitenabschnitts (22b) verläuft.

8. Inkontinenzwegwerfwindel nach mindestens einem der vorgenannten Ansprüche **dadurch gekennzeichnet, dass** die zweite (56a) und die dritte Faltanordnung (56b) zumindest bereichsweise zur Bildung eines Überlappungsbereiches (57) miteinander überlappen.

9. Inkontinenzwegwerfwindel nach Anspruch 8, wobei die zweite Faltanordnung (56a) in dem Überlappungsbereich (57) zwischen der dritten Faltanordnung (56b) und einer körperzugewandten Oberseite (4a) des Hauptteils (4) angeordnet ist.

10. Inkontinenzwegwerfwindel nach mindestens einem der vorgenannten Ansprüche, wobei zumindest die zweite Faltanordnung (56a) die Längsmittelachse LM zumindest bereichsweise überfängt.

11. Inkontinenzwegwerfwindel nach mindestens einem der vorgenannten Ansprüche, wobei eine jeweilige in der Querrichtung (10) innerste, das heißt der Längsmittelachse LM nächstgelegene in der Längsrichtung (8) verlaufende erste Produktfalzachse (70) und eine jeweilige in der Querrichtung (10) innerste, das heißt der Längsmittelachse LM nächstgelegene in der Längsrichtung (8) verlaufende zweite Produktfalzachse (71a) in einem gleichen Abstand A zur Längsmittelachse LM, insbesondere spiegelsymmetrisch zueinander mit der Längsmittelachse LM als Symmetrieachse angeordnet sind.

12. Inkontinenzwegwerfwindel nach mindestens einem der Ansprüche 8 bis 11, wobei der Hauptteil (4) beidseits zumindest abschnittsweise eine seitliche Auslaufsperre bildende und beidseits entlang der Längsrichtung (8) verlaufende Cuffelemente (23) aufweist, und wobei der von der zweiten (56a) und dritten Faltanordnung (56b) ausgebildete Überlappungsbereich (57) vollständig zwischen den Cuffelementen (23), mithin in einem Cuffelement-freien Bereich (100) angeordnet ist.

13. Inkontinenzwegwerfwindel nach mindestens einem der vorgenannten Ansprüche **dadurch gekennzeichnet, dass** die Inkontinenzwegwerfwindel eine maximale Spannweite MS aufweist, wobei die maximale Spannweite MS 500-1300 mm, insbesondere 600-1200 mm, weiter insbesondere 650-1000 mm, weiter insbesondere 700-900 mm beträgt.

14. Inkontinenzwegwerfwindel nach Anspruch 13 **dadurch gekennzeichnet, dass** die Inkontinenzwegwerfwindel eine Endfaltbreite EF aufweist und ein Verhältnis EF zu MS 0,15-0,35, insbesondere 0,17-0,30 beträgt.

15. Inkontinenzwegwerfwindel nach Anspruch 13 oder 14, **dadurch gekennzeichnet, dass** die Inkontinenzwegwerfwindel mit erster Faltanordnung (55a,55b) eine Spannweite GF1 aufweist und ein Verhältnis GF1 zu MS 0,50-0,85, insbesondere 0,55-0,75, weiter insbesondere 0,60-0,70 beträgt.

16. Inkontinenzwegwerfwindel nach einem oder mehreren der vorgenannten Ansprüche 13 bis 15 **dadurch gekennzeichnet, dass** ein Verhältnis der maximalen Spannweite MS der Inkontinenzwegwerfwindel zu einer vorderen Spannweite VS der Inkontinenzwegwerfwindel 1,3 - 3,5, insbesondere 1,5 - 3,2, weiter insbesondere 1,8 - 3,0, weiter insbesondere 2,0 - 2,8 beträgt.

17. Inkontinenzwegwerfwindel nach mindestens einem der vorgenannten Ansprüche, **dadurch gekennzeichnet, dass** die Inkontinenzwegwerfwindel um wenigstens eine, insbesondere um wenigstens zwei, weiter insbesondere um wenigstens drei in der Querrichtung (10) verlaufende Querfalzachsen (60) zu einer vierten Faltanordnung gefaltet ist, wobei keine der Querfalzachsen (60) durch den ersten (22a) oder zweiten hinteren Seitenabschnitt (22b) verläuft, insbesondere keine der Querfalzachsen (60) durch die jeweiligen Verschlussmittel (28) verläuft.

18. Inkontinenzwegwerfwindel nach mindestens einem der vorgenannten Ansprüche **dadurch gekennzeichnet, dass** der Saugkörper (6) der Inkontinenzwegwerfwindel eine maximale Quererstreckung in dem Rückenbereich (16) von QR aufweist und eine minimale Quererstreckung in dem Schrittbereich (17) von QS aufweist, wobei gilt 0,9 < QR/QS < 3,0.

19. Inkontinenzwegwerfwindel nach mindestens einem der vorgenannten Ansprüche **dadurch gekennzeichnet, dass** der jeweilige in der Querrichtung (10) elastische Bereich (42) des ersten (22a) oder zweiten hinteren Seitenabschnitts (22b) der Inkontinenzwegwerfwindel durch jeweils eine elastische Komponente mit einer jeweiligen maximalen Erstreckung QE in der Querrichtung (10) gebildet ist, wobei ein Verhältnis der maximalen Erstreckung QE der elastischen Komponenten des ersten (22a) oder zweiten hinteren Seitenabschnitts (22b) zu einer maximalen Erstreckung C des jeweiligen hinteren Seitenabschnitts (22a,22b) der Inkontinenzwegwerfwindel in der Längsrichtung (8) jeweils 0,30-0,70, insbesondere 0,40-0,60, weiter insbesondere 0,45-0,55, weiter insbesondere 0,53-0,43 beträgt.

20. Inkontinenzwegwerfwindel nach Anspruch 19, wobei ein Verhältnis der jeweiligen maximalen Erstreckung C des ersten hinteren Seitenabschnitts (22a) der Inkontinenzwegwerfwindel zu der jeweiligen maximalen Erstreckung C des zweiten hinteren Seitenabschnitts (22b) der Inkontinenzwegwerfwindel 0,9-1,1, insbesondere 1,0 beträgt.

21. Inkontinenzwegwerfwindel nach mindestens einem der vorgenannten Ansprüche, wobei die Inkontinenzwegwerfwindel derart ausgebildet ist, dass im an einen Nutzer angelegten Zustand ein jeweiliger elastischer Bereich (42) zumindest bereichsweise, eine Lücke P zwischen einem jeweiligen ersten (14a) oder zweiten vorderen seitlichen Längsrand (14b) des Vorderbereichs (12) und einem jeweiligen ersten (18a) oder zweiten hinteren seitlichen Längsrand (18b) des Rückenbereichs (16) überbrückt.

22. Inkontinenzwegwerfwindel nach mindestens einem der vorgenannten Ansprüche, wobei der erste (22a) und der zweite hintere Seitenabschnitt (22b) jeweils in der Querrichtung (10) einen an den jeweiligen ersten (18a) oder zweiten hinteren seitlichen Längsrand (18b) anschließenden proximalen Abschnitt (38) und einen an den proximalen Abschnitt (38) anschließenden, das freie Ende (41) des jeweiligen ersten (22a) oder zweiten hinteren Seitenabschnitts (22b) umfassenden frei endenden distalen Abschnitt (40) umfassen,

a) wobei der proximale Abschnitt (38) sich in Querrichtung (10) ausgehend von dem jeweiligen ersten (18a) oder zweiten hinteren seitlichen Längsrand (18b) über eine Länge erstreckt, die 65% der Erstreckung (44) des jeweiligen ersten (22a) oder zweiten hinteren Seitenabschnitts (22b) in der Querrichtung (10) beträgt,
b) wobei die in der Querrichtung (10) elastische Komponente des ersten (22a) und zweiten hinteren Seitenabschnitts (22b) jeweils zumindest bereichsweise, insbesondere vollständig innerhalb des jeweiligen proximalen Abschnitts (38) angeordnet ist,
c) wobei der erste (22a) und der zweite hintere Seitenabschnitt (22b) jeweils zumindest bereichsweise, insbesondere in dem gesamten jeweiligen distalen Abschnitt (40) in der Querrichtung (10) im Wesentlichen undehnbar ausgebildet sind.

**Claims**

1. Disposable incontinence nappy having a longitudinal direction (8), a transverse direction (10), a longitudinal central axis LM, and a main part (4), comprising an absorbent body (6), wherein the main part (4) has a front region (12) having a first (14a) and a second front lateral longitudinal edge (14b) arranged opposite in the transverse direction (10), and a back region (16) having a first (18a) and a second rear lateral longitudinal edge (18b) arranged opposite in the transverse direction (10), and wherein the main part (4) has a crotch region (17) arranged between the front region (12) and the back region (16), and wherein the main part (4) has a first rear side section (22a) and a second rear side section (22b), wherein the first rear side section (22a) is joined to the first rear lateral longitudinal edge (18a), and wherein the second rear side section (22b) is joined to the second rear lateral longitudinal edge (18b), wherein the first (22a) and the second rear side section (22b) each have a region (42) which is elastic in the transverse direction (10), wherein the first (22a) and the second rear side section (22b) each have at least one closure means (28), thus making it possible, for the sake of applying the disposable incontinence nappy to a user and closing said nappy, to wrap the first (22a) and the second rear side section (22b) respectively around the body of the user along a hip circumferential direction and bring them into overlapping arrangement with an outer side of the front region (12), to which they can each be releasably attached by means of the respective closure means (28),

   a) wherein the first rear side section (22a) is folded onto itself at a number N1 of first side section folding axes (50a, 51a) extending in the longitudinal direction (8) to form a first fold arrangement of the first rear side section (55a),
   b) wherein the second rear side section (22b) is folded onto itself at a number N2 of second side section folding axes (50b, 51b) extending in the longitudinal direction (8) to form a first fold arrangement of the second rear side section (55b),
   c) wherein the first fold arrangement of the first rear side section (55a) is folded inward onto a body-facing upper side (4a) of the main part (4) at a number N3 of first product folding axes (70) extending in the longitudinal direction (8) to form a second fold arrangement (56a),
   d) wherein the first fold arrangement (55b) of the second rear side section (22b) is folded inward onto the body-facing upper side (4a) of the main part (4) at a number N4 of second product folding axes (71a, 71b) extending in the longitudinal direction (8) to form a third fold arrangement (56b),
   e) wherein N3<N4.

2. Disposable incontinence nappy according to Claim 1, **characterized in that** N3$\geq$1, N4$\leq$4, in particular N3=1 and N4$\geq$2, wherein, in particular, N4=2.

3. Disposable incontinence nappy according to at least one of the preceding claims, **characterized in that** the number N1 of the first side section folding axes (50a, 51a) which extend in the longitudinal direction (8) and the number N2 of the second side section folding axes (50b, 51b) which extend in the longitudinal direction (8) is in each case 1-5, in particular 1-4, and more particularly 1-3.

4. Disposable incontinence nappy according to at least one of the preceding claims, wherein N1=N2, wherein in particular N1=2, wherein more particularly the first fold arrangement of the first rear side section (55a) and the first fold arrangement of the second rear side section (55b) are each of Z-shaped design.

5. Disposable incontinence nappy according to at least one of the preceding claims, wherein the at least one closure means (28) of the first (22a) and/or second rear side section (22b) is arranged at a free end (41) of the respective first (22a) or second rear side section (22b).

6. Disposable incontinence nappy according to at least one of the preceding claims, wherein, in the first fold arrangement (55a, 55b), the at least one closure means (28) of the first (22a) and/or second rear side section (22b) comes to lie outside the respective first (18a) or second rear lateral longitudinal edge (18b) of the back region (16), that is to say outside the main part (4).

7. Disposable incontinence nappy according to at least one of the preceding claims, wherein a second product folding axis (70), which is outermost in the transverse direction (10) and extends in the longitudinal direction (8), extends outside the absorbent body (6), in particular inside the second rear side section (22b), more particularly inside the second rear side section (22b) and outside the first fold arrangement (55b) of the second rear side section (22b).

8. Disposable incontinence nappy according to at least one of the preceding claims, **characterized in that** the second (56a) and the third fold arrangement (56b) overlap each other at least in some region or regions to form an overlap region (57).

9. Disposable incontinence nappy according to Claim 8, wherein the second fold arrangement (56a) is arranged in the overlap region (57) between the third fold arrangement (56b) and a body-facing upper side (4a) of the main part (4).

10. Disposable incontinence nappy according to at least one of the preceding claims, wherein at least the second fold arrangement (56a) overlaps the longitudinal central axis LM, at least in some region or regions.

11. Disposable incontinence nappy according to at least one of the preceding claims, wherein a respective first product folding axis (70), which is innermost in the transverse direction (10), that is to say is closest to the longitudinal central axis LM, and extends in the longitudinal direction (8), and a respective second product folding axis (71a), which is innermost in the transverse direction (10), that is to say is closest to the longitudinal central axis LM, and extends in the longitudinal direction (8), are arranged at the same distance A from the longitudinal central axis LM, in particular mirror-symmetrically with respect to one another, with the longitudinal central axis LM as the axis of symmetry.

12. Disposable incontinence nappy according to at least one of Claims 8 to 11, wherein the main part (4) has cuff elements (23) on both sides, which form a lateral leakage barrier at least in some section or sections and extend along the longitudinal direction (8) on both sides, and wherein the overlap region (57) formed by the second (56a) and third fold arrangements (56b) is arranged completely between the cuff elements (23), and thus in a region (100) free of cuff elements.

13. Disposable incontinence nappy according to at least one of the preceding claims, **characterized in that** the disposable incontinence nappy has a maximum span MS, wherein the maximum span MS is 500-1300 mm, in particular 600-1200 mm, more particularly 650-1000 mm, more particularly 700-900 mm.

14. Disposable incontinence nappy according to Claim 13, **characterized in that** the disposable incontinence nappy has a final folded width EF, and a ratio EF to MS is 0.15-0.35, in particular 0.17-0.30.

15. Disposable incontinence nappy according to Claim 13 or 14, **characterized in that** the disposable incontinence nappy with the first fold arrangement (55a, 55b) has a span GF1, and a ratio GF1 to MS is 0.50-0.85, in particular 0.55-0.75, more particularly 0.60-0.70.

16. Disposable incontinence nappy according to one or more of the preceding Claims 13 to 15, **characterized in that** a ratio of the maximum span MS of the disposable incontinence nappy to a front span VS of the disposable incontinence nappy is 1.3-3.5, in particular 1.5-3.2, more particularly 1.8-3.0, more particularly 2.0-2.8.

17. Disposable incontinence nappy according to at least one of the preceding claims, **characterized in that** the disposable incontinence nappy is folded about at least one, in particular about at least two, more particularly about at least three transverse folding axes (60) extending in the transverse direction (10) to form a fourth fold arrangement, wherein none of the transverse folding axes (60) extends through the first (22a) or second rear side section (22b), in particular none of the transverse folding axes (60) extends through the respective closure means (28).

18. Disposable incontinence nappy according to at least one of the preceding claims, **characterized in that** the absorbent body (6) of the disposable incontinence nappy has a maximum transverse extent in the back region (16) of QR and has a minimum transverse extent in the crotch region (17) of QS, wherein $0.9 < QR/QS < 3.0$.

19. Disposable incontinence nappy according to at least one of the preceding claims, **characterized in that** the respective region (42), which is elastic in the transverse direction (10), of the first (22a) or second rear side section (22b) of the disposable incontinence nappy is formed in each case by an elastic component having a respective maximum extent QE in the transverse direction (10), wherein a ratio of the maximum extent QE of the elastic components of the first (22a) or second rear side section (22b) to a maximum extent C of the respective rear side section (22a, 22b) of the disposable incontinence nappy in the longitudinal direction (8) is in each case 0.30-0.70, in particular 0.40-0.60, more particularly 0.45-0.55, more particularly 0.53-0.43.

**20.** Disposable incontinence nappy according to Claim 19, wherein a ratio of the respective maximum extent C of the first rear side section (22a) of the disposable incontinence nappy to the respective maximum extent C of the second rear side section (22b) of the disposable incontinence nappy is 0.9-1.1, in particular 1.0.

**21.** Disposable incontinence nappy according to at least one of the preceding claims, wherein the disposable incontinence nappy is designed in such a way that, in the state applied to a user, a respective elastic region (42) bridges, at least in some region or regions, a gap P between a respective first (14a) or second front lateral longitudinal edge (14b) of the front region (12) and a respective first (18a) or second rear lateral longitudinal edge (18b) of the back region (16).

**22.** Disposable incontinence nappy according to at least one of the preceding claims, wherein the first (22a) and the second rear side section (22b) each comprise, in the transverse direction (10), a proximal section (38), which adjoins the respective first (18a) or second rear lateral longitudinal edge (18b), and a free-ending distal section (40), which adjoins the proximal section (38) and comprises the free end (41) of the respective first (22a) or second rear side section (22b),

a) wherein the proximal section (38) extends in the transverse direction (10), starting from the respective first (18a) or second rear lateral longitudinal edge (18b), over a length which is 65% of the extent (44) of the respective first (22a) or second rear side section (22b) in the transverse direction (10),
b) wherein the component of the first (22a) and second rear side sections (22b) which is elastic in the transverse direction (10) is in each case arranged inside the respective proximal section (38), at least in some region or regions, in particular completely,
c) wherein the first (22a) and the second rear side section (22b) are each designed to be substantially non-stretchable in the transverse direction (10), at least in some region or regions, in particular in the entire respective distal section (40).

**Revendications**

**1.** Couche d'incontinence jetable comprenant une direction longitudinale (8), une direction transversale (10), un axe central longitudinal LM, et une partie principale (4) comprenant un corps absorbant (6), la partie principale (4) comportant une zone avant (12) pourvue d'un premier bord longitudinal latéral avant (14a) et d'un deuxième bord longitudinal latéral avant (14b) opposé dans la direction transversale (10) et une zone arrière (16) pourvue d'un premier bord longitudinal latéral arrière (18a) et d'un deuxième bord longitudinal latéral arrière (18b) opposé dans la direction transversale (10), et la partie principale (4) comportant une zone d'entrejambe (17) disposée entre la zone avant (12) et la zone arrière (16), et la partie principale (4) comportant une première portion latérale arrière (22a) et une deuxième portion latérale arrière (22b), la première portion latérale arrière (22a) étant reliée au premier bord longitudinal latéral arrière (18a), et la deuxième portion latérale arrière (22b) étant reliée au deuxième bord longitudinal latéral arrière (18b), les première (22a) et deuxième portions latérales arrière (22b) comportant chacune une zone (42) élastique dans la direction transversale (10), les première (22a) et deuxième (22b) portions latérales arrière comportant chacune au moins un moyen de fermeture (28) de sorte que les première (22a) et deuxième (22b) portions latérales arrière peuvent être placées chacune autour du corps d'un utilisateur dans la direction périphérique des hanches afin de mettre et fermer la couche d'incontinence jetable sur l'utilisateur et être amenées dans un agencement de chevauchement avec un côté extérieur de la zone avant (12) auquel elles peuvent chacune être collées de manière amovible par le biais du moyen de fermeture respectif (28),

a) la première portion latérale arrière (22a) étant repliée sur elle-même sur un nombre N1 de premiers axes de pliage de portion latérale (50a, 51a) qui s'étendent dans la direction longitudinale (8) dans un premier agencement de pliage de la première portion latérale arrière (55a),
b) la deuxième portion latérale arrière (22b) étant repliée sur elle-même sur un nombre N2 de deuxièmes axes de pliage de portion latérale (50b, 51b) qui s'étendent dans la direction longitudinale (8) dans un premier agencement de pliage de la deuxième portion latérale arrière (55a),
c) le premier agencement de pliage de la première portion latérale arrière (55a) étant plié vers l'intérieur sur un côté supérieur (4a), dirigé vers le corps, de la partie principale (4) sur un nombre N3 de premiers axes de pliage de produit (70) qui s'étendent dans la direction longitudinale (8) dans un deuxième agencement de pliage (56a),
d) le premier agencement de pliage (55b) de la deuxième portion latérale arrière (22b) étant plié vers l'intérieur sur un côté supérieur (4a), dirigé vers le corps, de la partie principale (4) sur un nombre N4 de deuxièmes axes de pliage de produit (71a, 71b) qui s'étendent dans la direction longitudinale (8) dans un troisième agencement de pliage (56b),
e) avec N3 < N4.

2. Couche d'incontinence jetable selon la revendication 1, **caractérisée en ce que** N3 ≥ 1, N4 ≤ 4, notamment N3 = 1 et N4 ≥ 2, avec en particulier N4 = 2.

3. Couche d'incontinence jetable selon l'une au moins des revendications précédentes, **caractérisée en ce que** le nombre N1 des premiers axes de pliage de portion latérale (50a, 51a) qui s'étendent dans la direction longitudinale (8) et le nombre N2 des deuxièmes axes de pliage de portion latérale (50b, 51b) qui s'étendent dans la direction longitudinale (8) vont respectivement de 1 à 5, en particulier de 1 à 4, plus particulièrement de 1 à 3.

4. Couche d'incontinence jetable selon l'une au moins des revendications précédentes, avec N1 = N2, notamment N1 = 2, plus particulièrement le premier agencement de pliage de la première portion latérale arrière (55a) et le premier agencement de pliage de la deuxième portion latérale arrière (55b) étant chacun en forme de Z.

5. Couche d'incontinence jetable selon l'une au moins des revendications précédentes, l'au moins un moyen de fermeture (28) des première (22a) et/ou deuxième (22b) portions latérales arrière étant disposé à une extrémité libre (41) de la première (22a) ou deuxième (22b) portion latérale arrière respective.

6. Couche d'incontinence jetable selon l'une au moins des revendications précédentes, l'au moins un moyen de fermeture (28) des première (22a) et/ou deuxième (22b) portions latérales arrière dans le premier agencement de pliage (55a, 55b) venant se placer à l'extérieur du premier (18a) ou deuxième (18b) bord longitudinal latéral arrière respectif de la zone arrière (16), c'est-à-dire à l'extérieur de la partie principale (4).

7. Couche d'incontinence jetable selon l'une au moins des revendications précédentes, un deuxième axe de pliage de produit (70) qui s'étend dans la direction longitudinale (8) le plus à l'extérieur dans la direction transversale (10) s'étendant à l'extérieur du corps absorbant (6), en particulier à l'intérieur de la deuxième portion latérale arrière (22b), plus particulièrement à l'intérieur de la deuxième portion latérale arrière (22b) et à l'extérieur du premier agencement de pliage (55b) de la deuxième portion latérale arrière (22b).

8. Couche d'incontinence jetable selon l'une au moins des revendications précédentes, **caractérisée en ce que** le deuxième (56a) et le troisième agencement de pliage (56b) se chevauchent au moins par zones pour former une zone de chevauchement (57).

9. Couche d'incontinence jetable selon la revendication 8, le deuxième agencement de pliage (56a) étant disposé dans la zone de chevauchement (57) située entre le troisième agencement de pliage (56b) et un côté supérieur (4a), dirigé vers le corps, de la partie principale (4).

10. Couche d'incontinence jetable selon l'une au moins des revendications précédentes, au moins le deuxième agencement de pliage (56a) venant au moins partiellement par-dessus l'axe central longitudinal LM.

11. Couche d'incontinence jetable selon l'une au moins des revendications précédentes, un premier axe de pliage de produit respectif (70) qui s'étend dans la direction longitudinale (8) le plus à l'intérieur dans la direction transversale (10), c'est-à-dire le plus proche de l'axe central longitudinal LM, et un deuxième axe de pliage de produit respectif (71a) qui s'étend dans la direction longitudinale (8) le plus à l'intérieur dans la direction transversale (10), c'est-à-dire le plus proche de l'axe central longitudinal LM, étant disposés à égale distance A de l'axe central longitudinal LM, en particulier en symétrie de miroir l'un par rapport à l'autre avec l'axe central longitudinal LM comme axe de symétrie.

12. Couche d'incontinence jetable selon l'une au moins des revendications 8 à 11, la partie principale (4) comportant des éléments de revers (23) qui forment des deux côtés au moins par portions une barrière anti-fuites latérale et qui s'étendent des deux côtés le long de la direction longitudinale (8), et la zone de chevauchement (57) formée par les deuxième (56a) et troisième (56b) agencements de pliage étant disposée entièrement entre les éléments de revers (23), par conséquent dans une zone sans élément de revers (100).

13. Couche d'incontinence jetable selon l'une au moins des revendications précédentes, **caractérisée en ce que** la couche d'incontinence jetable a une portée maximale MS, la portée maximale MS étant de 500 à 1300 mm, notamment de 600 à 1200 mm, plus particulièrement de 650 à 1000 mm, plus particulièrement de 700 à 900 mm.

**14.** Couche d'incontinence jetable selon la revendication 13, **caractérisée en ce que** la couche d'incontinence jetable a une largeur de pliage finale EF et un rapport EF à MS est de 0,15 à 0,35, en particulier de 0,17 à 0,30.

**15.** Couche d'incontinence jetable selon la revendication 13 ou 14, **caractérisée en ce que** la couche d'incontinence jetable pourvue d'un premier agencement de pliage (55a, 55b) a une portée GF1 et un rapport GF1 sur MS est de 0,50 à 0,85, en particulier de 0,55 à 0,75, plus particulièrement de 0,60 à 0,70.

**16.** Couche d'incontinence jetable selon une ou plusieurs des revendications précédentes 13 à 15, **caractérisée en ce qu'**un rapport de la portée maximale MS de la couche d'incontinence jetable à une portée avant VS de la couche d'incontinence jetable est de 1,3 à 3,5, en particulier de 1,5 à 3,2, en particulier de 1,8 à 3,0, plus particulièrement de 2,0 à 2,8.

**17.** Couche d'incontinence jetable selon l'une au moins des revendications précédentes, **caractérisée en ce que** la couche d'incontinence jetable est pliée autour d'au moins un, notamment d'au moins deux, plus particulièrement d'au moins trois, axes de pliage transversaux (60) qui s'étendent dans la direction transversale (10) pour former un quatrième agencement de pliage, aucun des axes de pliage transversaux (60) ne s'étendant à travers la première (22a) ou deuxième (22b) portion latérale arrière, en particulier aucun des axes de pliage transversaux (60) ne s'étendant à travers les moyens de fermeture respectifs (28).

**18.** Couche d'incontinence jetable selon l'une au moins des revendications précédentes, **caractérisée en ce que** le corps absorbant (6) de la couche d'incontinence jetable a une étendue transversale maximale dans la zone arrière (16) de QR et une étendue transversale minimale dans la zone d'entrejambe (17) de QS, avec 0,9 <QR/QS < 3,0.

**19.** Couche d'incontinence jetable selon l'une au moins des revendications précédentes, **caractérisée en ce que** la zone respective (42), élastique dans la direction transversale (10), de la première (22a) ou deuxième (22b) portion latérale arrière de la couche d'incontinence jetable est formée par un composant élastique ayant une étendue maximale respective QE dans la direction transversale (10), un rapport de l'étendue maximale QE des composants élastiques de la première (22a) ou deuxième (22b) portion latérale arrière à une étendue maximale C de la portion latérale arrière respective (22a, 22b) de la couche d'incontinence jetable dans la direction longitudinale (8) étant de 0,30 à 0,70, en particulier de 0,40 à 0,60, plus particulièrement de 0,45 à 0,55, plus particulièrement de 0,53 à 0,43.

**20.** Couche d'incontinence jetable selon la revendication 19, un rapport de l'étendue maximale respective C de la première portion latérale arrière (22a) de la couche d'incontinence jetable à l'étendue maximale respective C de la deuxième portion latérale arrière (22b) de la couche d'incontinence jetable étant de 0,9 à 1,1, en particulier de 1.0.

**21.** Couche d'incontinence jetable selon l'une au moins des revendications précédentes, la couche d'incontinence jetable étant conçue de manière à ce que, lorsqu'elle est mise sur un utilisateur, une zone élastique respective (42) couvre au moins par zones un espace P ménagé entre un premier (14a) ou deuxième bord longitudinal latéral avant respectif (14b) de la zone avant (12) et un premier (18a) ou deuxième bord longitudinal latéral arrière respectif (18b) de la zone arrière (16) .

**22.** Couche d'incontinence jetable selon l'une au moins des revendications précédentes, les première (22a) et deuxième (22b) portions latérales arrière comprenant chacune dans la direction transversale (10) une portion proximale (38) attenante au premier (18a) ou deuxième (18b) bord longitudinal latéral arrière respectif et une portion distale (40) à extrémité libre qui est attenante à la portion proximale (38) et qui comprend l'extrémité libre (41) de la première (22a) ou deuxième (22b) portion latérale arrière respective,

a) la portion proximale (38) s'étendant dans la direction transversale (10) depuis le premier (18a) ou deuxième (18b) bord longitudinal latéral arrière respectif sur une longueur qui est égale à 65 % de l'étendue (44) de la première (22a) ou deuxième (22b) portion latérale arrière respective dans la direction transversale (10),
b) le composant élastique dans la direction transversale (10) des première (22a) et deuxième (22b) portions latérales arrière étant à chaque fois disposé au moins par zones, en particulier entièrement, à l'intérieur de la portion proximale respective (38),
c) les première (22a) et deuxième (22b) portions latérales arrière étant chacune conçues pour être sensiblement inextensibles dans la direction transversale (10) au moins par zones, en particulier dans toute la portion distale respective (40).

Fig. 1

Fig. 2

EP 3 858 310 B1

Fig. 3a

Fig. 3b

Fig. 3d

Fig. 3c

Fig. 3e

Fig. 4

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2017140604 A1
- WO 2005110321 A1
- EP 0650714 A1